# EUROPEAN PATENT APPLICATION

(11) **EP 3 456 824 A1**
(43) Date of publication of application: **20.03.2019**
(21) Application number: 17796259.4
(22) Date of filing: 12.05.2017
(51) Int. Cl.: C12N 15/09, A61K 39/395, A61K 45/00, A61P 1/16, A61P 3/00, A61P 3/04, A61P 3/10, A61P 5/50, A61P 29/00, A61P 43/00, C07K 16/18, C12N 15/113, C12P 19/34, C12Q 1/68

(54) **OBESITY-RELATED DISEASE THERAPEUTIC AGENT BY HEPATIC SECRETORY METABOLIC REGULATOR INHIBITORY ACTION**

(30) Priority: 13.05.2016 JP 2016097233
(71) Applicant: The University of Tokyo, Bunkyo-ku Tokyo 113-8654 (JP)
(72) Inventor: IZUMIDA, Yoshihiko, Tokyo 113-8654 (JP); WADA, Nobuhiro, Tokyo 113-8654 (JP); MASUDA, Yukari, Tokyo 113-8654 (JP); KADOWAKI, Takashi, Tokyo 113-8654 (JP)
(74) Representative: Regimbeau
(86) International application number: PCT/JP2017/018093
(87) International publication number: WO 2017/195901

(57) **Abstract**

Disclosed are methods for screening a drug for treating obesity-related diseases by using the ability of candidate drugs to inhibit the function of pentraxin-4 as an index, and a pharmaceutical composition for treating obesity-related diseases comprising a pentraxin-4 inhibitor as an active ingredient. The drug screened by the above-mentioned method and the pharmaceutical composition are useful for treating obesity-related diseases, particularly for non-alcoholic steatohepatitis (NASH).

SEQUENCE LISTING

<110>  The University of Tokyo

<120>  Pharmaceutical Composition for Treatment of Obesity-related
       Diseases or Disorders comprising Inhibitor of Liver
       Secreted-type Metabolism Regulatory Factor

<130>  218925

<160>  41

<210>  1
<211>  53
<212>  DNA
<213>  Artificial Sequence

<220>
<221>
<222>
<223>  Desined nucleotide for expressing RNAi mediator

<400>  1

tgataataag ttagtactaa cgtgtgctgt ccgtcagtac taacttgttg tca        53

<210>  2
<211>  54
<212>  DNA
<213>  Artificial Sequence

<220>
<221>
<222>
<223>  Desined nucleotide for expressing RNAi mediator

<400>  2

cactattatt caatcatgat tgcacacgac aggcagtcat gattgaacaa cagt       54

<210>  3
<211>  15
<212>  PRT
<213>  Artificial Sequence

<220>
<221>
<222>
<223>  Desined polypeptide as antigen

<400>  3

Cys Gln Val Ala Lys Ser Ser Pro Gln His Arg Ser Ser Pro His
1               5                   10                  15

<210>  4
<211>  14
<212>  PRT
<213>  Artificial Sequence

<220>
<221>
<222>

<223>  Desined polypeptide as antigen

<400>  4

Cys Glu Gln Asp Thr Val Gly Gly Glu Phe Asp Ser Ser Glu
1               5                   10

<210>  5
<211>  14
<212>  PRT
<213>  Artificial Sequence

<220>
<221>
<222>
<223>  Desined polypeptide as antigen

<400>  5

Cys Glu Gln Asp Ser Val Gly Gly Gly Phe Asp Ser Ser Glu
1               5                   10

<210>  6
<211>  19
<212>  DNA
<213>  Artificial Sequence

<220>
<221>
<222>
<223>  Designed primer for quantitative RT-PCR

<400>  6

gtcactgtcc acgttcagg                                                    19

<210>  7
<211>  20
<212>  DNA
<213>  Artificial Sequence

<220>
<221>
<222>
<223>  Designed primer for quantitative RT-PCR

<400>  7

gtccttggta ggtccttggt                                                   20

<210>  8
<211>  25
<212>  DNA
<213>  Artificial Sequence

<220>
<221>
<222>
<223>  Designed primer for quantitative RT-PCR

<400>  8

ggaagaaact ctatgacggg ttatt                                             25

<210>  9
<211>  23
<212>  DNA
<213>  Artificial Sequence

<220>
<221>
<222>
<223>  Designed primer for quantitative RT-PCR

<400>  9

catcgatcat attgtgagtg gtc                                              23

<210>  10
<211>  22
<212>  DNA
<213>  Artificial Sequence

<220>
<221>
<222>
<223>  Designed primer for quantitative RT-PCR

<400>  10

ggacattatc accttgtttg gc                                               22

<210>  11
<211>  22
<212>  DNA
<213>  Artificial Sequence

<220>
<221>
<222>
<223>  Designed primer for quantitative RT-PCR

<400>  11

ggagcaacac ctattcattt gg                                               22

<210>  12
<211>  26
<212>  DNA
<213>  Artificial Sequence

<220>
<221>
<222>
<223>  Designed primer for quantitative RT-PCR

<400>  12

gacctcatca aagatactct cctgaa                                           26

<210>  13
<211>  24
<212>  DNA
<213>  Artificial Sequence

<220>

<221>
<222>
<223>  Designed primer for quantitative RT-PCR

<400>  13

atctcgtctt gaccacatca acag                                              24

<210>  14
<211>  20
<212>  DNA
<213>  Artificial Sequence

<220>
<221>
<222>
<223>  Designed primer for quantitative RT-PCR

<400>  14

aggatggtga acccgacaac                                                   20

<210>  15
<211>  20
<212>  DNA
<213>  Artificial Sequence

<220>
<221>
<222>
<223>  Designed primer for quantitative RT-PCR

<400>  15

ttggatctga aggcggactt                                                   20

<210>  16
<211>  22
<212>  DNA
<213>  Artificial Sequence

<220>
<221>
<222>
<223>  Designed primer for quantitative RT-PCR

<400>  16

aacctgctgg tgtgtgacgt tc                                                22

<210>  17
<211>  22
<212>  DNA
<213>  Artificial Sequence

<220>
<221>
<222>
<223>  Designed primer for quantitative RT-PCR

<400>  17

cagcacgagg ctttttgtt gt                                                 22

<210>  18
<211>  22
<212>  DNA
<213>  Artificial Sequence

<220>
<221>
<222>
<223>  Designed primer for quantitative RT-PCR

<400>  18

tgtggagatc atcgaacagc cg                                              22

<210>  19
<211>  25
<212>  DNA
<213>  Artificial Sequence

<220>
<221>
<222>
<223>  Designed primer for quantitative RT-PCR

<400>  19

ttcctggtcc tgtgtagcca ttgat                                           25

<210>  20
<211>  20
<212>  DNA
<213>  Artificial Sequence

<220>
<221>
<222>
<223>  Designed primer for quantitative RT-PCR

<400>  20

ttccagctga ggaaagtgtg                                                 20

<210>  21
<211>  20
<212>  DNA
<213>  Artificial Sequence

<220>
<221>
<222>
<223>  Designed primer for quantitative RT-PCR

<400>  21

agctgttatt ccggaggaga                                                 20

<210>  22
<211>  15
<212>  DNA
<213>  Artificial Sequence

<220>

<221>
<222>
<223>  Designed primer for quantitative RT-PCR

<400>  22

cggcgcggaa gctgt                                                        15

<210>  23
<211>  18
<212>  DNA
<213>  Artificial Sequence

<220>
<221>
<222>
<223>  Designed primer for quantitative RT-PCR

<400>  23

tgcaatccat ggctccgt                                                     18

<210>  24
<211>  20
<212>  DNA
<213>  Artificial Sequence

<220>
<221>
<222>
<223>  Designed primer for quantitative RT-PCR

<400>  24

ctgcagcctc aagtgcaaag                                                   20

<210>  25
<211>  20
<212>  DNA
<213>  Artificial Sequence

<220>
<221>
<222>
<223>  Designed primer for quantitative RT-PCR

<400>  25

cagtgtgcca ttggctgtct                                                   20

<210>  26
<211>  21
<212>  DNA
<213>  Artificial Sequence

<220>
<221>
<222>
<223>  Designed primer for quantitative RT-PCR

<400>  26

gccgtgttaa ggaatctgct g                                                 21

<210>  27
<211>  21
<212>  DNA
<213>  Artificial Sequence

<220>
<221>
<222>
<223>  Designed primer for quantitative RT-PCR

<400>  27

tgctcttctg tatcgcccag t                                                21

<210>  28
<211>  23
<212>  DNA
<213>  Artificial Sequence

<220>
<221>
<222>
<223>  Designed primer for quantitative RT-PCR

<400>  28

cagtgtggtg cacgtctcca atc                                              23

<210>  29
<211>  21
<212>  DNA
<213>  Artificial Sequence

<220>
<221>
<222>
<223>  Designed primer for quantitative RT-PCR

<400>  29

tgaaccaaag ttgaccacca g                                                21

<210>  30
<211>  20
<212>  DNA
<213>  Artificial Sequence

<220>
<221>
<222>
<223>  Designed primer for quantitative RT-PCR

<400>  30

gagcggagag tactggatcg                                                  20

<210>  31
<211>  20
<212>  DNA
<213>  Artificial Sequence

<220>

<221>
<222>
<223>  Designed primer for quantitative RT-PCR

<400>  31

gttcgggctg atgtaccagt                                                    20

<210>  32
<211>  20
<212>  DNA
<213>  Artificial Sequence

<220>
<221>
<222>
<223>  Designed primer for quantitative RT-PCR

<400>  32

aggctgaagg aaacagcaaa                                                    20

<210>  33
<211>  20
<212>  DNA
<213>  Artificial Sequence

<220>
<221>
<222>
<223>  Designed primer for quantitative RT-PCR

<400>  33

tagtctcatt gccttgcgtg                                                    20

<210>  34
<211>  20
<212>  DNA
<213>  Artificial Sequence

<220>
<221>
<222>
<223>  Designed primer for quantitative RT-PCR

<400>  34

gaagtgaaac ctcactcacg                                                    20

<210>  35
<211>  21
<212>  DNA
<213>  Artificial Sequence

<220>
<221>
<222>
<223>  Designed primer for quantitative RT-PCR

<400>  35

caataaagac tacagcaccc c                                                  21

<210>  36
<211>  20
<212>  DNA
<213>  Artificial Sequence

<220>
<221>
<222>
<223>  Designed primer for quantitative RT-PCR

<400>  36

taaggatttc ggtggtgaca                                                    20

<210>  37
<211>  20
<212>  DNA
<213>  Artificial Sequence

<220>
<221>
<222>
<223>  Designed primer for quantitative RT-PCR

<400>  37

tgtcaccacc gaaatcctta                                                    20

<210>  38
<211>  20
<212>  DNA
<213>  Artificial Sequence

<220>
<221>
<222>
<223>  Designed primer for quantitative RT-PCR

<400>  38

gaagacaggg cgacctggaa                                                    20

<210>  39
<211>  22
<212>  DNA
<213>  Artificial Sequence

<220>
<221>
<222>
<223>  Designed primer for quantitative RT-PCR

<400>  39

ttgtctgctc ccacaatgaa gc                                                 22

<210>  40
<211>  1422
<212>  DNA
<213>  Homo sapiens

<220>

<221>  CDS
<222>  (1) to (1419)
<223>

<400>  40

atg ggg agt gga aac tgg gag gtc aca ggt cca cct tgt ggc agt cgg     48
Met Gly Ser Gly Asn Trp Glu Val Thr Gly Pro Pro Cys Gly Ser Arg
1               5                   10                  15
tgt gaa cgg cct ccc gga ctc cca cgc ggg gtc cac gga cag ttc cgg     96
Cys Glu Arg Pro Pro Gly Leu Pro Arg Gly Val His Gly Gln Phe Arg
            20                  25                  30
agt gct gtg ggc ctg tcg ggc ctg cgc tgg ttc cgg aga ttc cag gag     144
Ser Ala Val Gly Leu Ser Gly Leu Arg Trp Phe Arg Arg Phe Gln Glu
        35                  40                  45
gtg acc tgg aca cac ctg cag aac atc gcc agc aac tac aac gtg tcc     192
Val Thr Trp Thr His Leu Gln Asn Ile Ala Ser Asn Tyr Asn Val Ser
    50                  55                  60
tac aac gtt gac gtc cgg ttc cgg agc ctg gcg gaa gag agc cag gct     240
Tyr Asn Val Asp Val Arg Phe Arg Ser Leu Ala Glu Glu Ser Gln Ala
65                  70                  75                  80
gtg gct cag gca gtc aac cgg tca cag gcc tcg gtg cag ggg gag ctg     288
Val Ala Gln Ala Val Asn Arg Ser Gln Ala Ser Val Gln Gly Glu Leu
                85                  90                  95
gcg cag ctc aag gcc tgg gtg agg aag ctg cag cgc cga ggc cgg aaa     336
Ala Gln Leu Lys Ala Trp Val Arg Lys Leu Gln Arg Arg Gly Arg Lys
            100                 105                 110
gta gac acg cgg ctg cgg gcc ttg gac ctc acc ctg ggc gag agg agc     384
Val Asp Thr Arg Leu Arg Ala Leu Asp Leu Thr Leu Gly Glu Arg Ser
        115                 120                 125
cag cag cgg gcc cgg gaa agg aag gca cac aag gcc cag agg gac gcc     432
Gln Gln Arg Ala Arg Glu Arg Lys Ala His Lys Ala Gln Arg Asp Ala
    130                 135                 140
ctg cag gac tca ctg gca cgc ctg gag ggc ctc gtc cac agc cag ggc     480
Leu Gln Asp Ser Leu Ala Arg Leu Glu Gly Leu Val His Ser Gln Gly
145                 150                 155                 160
gcc agg ctg gct gct ctg gag ggg cgg ctg ccc gtg gcc cac cct ggc     528
Ala Arg Leu Ala Ala Leu Glu Gly Arg Leu Pro Val Ala His Pro Gly
                165                 170                 175
act gca gcc ctc ggg ccg gcc ctg gtc cca acc cca acc cag cct gag     576
Thr Ala Ala Leu Gly Pro Ala Leu Val Pro Thr Pro Thr Gln Pro Glu
            180                 185                 190
gag ctg ggc ccg acc tcc ctg aag ctt cag agg gac agg cag gag ctc     624
Glu Leu Gly Pro Thr Ser Leu Lys Leu Gln Arg Asp Arg Gln Glu Leu
        195                 200                 205
cga gct gcc tct gag cac agg ggc ccc cca cag gac tcc tcg gcc cct     672
Arg Ala Ala Ser Glu His Arg Gly Pro Pro Gln Asp Ser Ser Ala Pro
    210                 215                 220
ctc caa ggg agg cgg gag cct cca gcc tca ggc agc cat cgg gta ctc     720
Leu Gln Gly Arg Arg Glu Pro Pro Ala Ser Gly Ser His Arg Val Leu
225                 230                 235                 240
agt ggg act gcc cca aaa gac cct cgg cag cag gca tgg tcc ccc cag     768
Ser Gly Thr Ala Pro Lys Asp Pro Arg Gln Gln Ala Trp Ser Pro Gln
                245                 250                 255
gtg cca gga gag att tgc ggc gtg ggc ccc acc ctc gtt ttc cca aac     816
Val Pro Gly Glu Ile Cys Gly Val Gly Pro Thr Leu Val Phe Pro Asn
            260                 265                 270
gcc tcc acc agg aac gtg gtc ttc ctc agc cct ggt ttc gtc act gcc     864
Ala Ser Thr Arg Asn Val Val Phe Leu Ser Pro Gly Phe Val Thr Ala
        275                 280                 285
ctg cga gcc ctg tcc ttc tgc agc tgg gtc cgc acg gcc tcc ggc cgc     912
Leu Arg Ala Leu Ser Phe Cys Ser Trp Val Arg Thr Ala Ser Gly Arg
    290                 295                 300

ctg ggc acc ctc ctg tcc tac gcc acc gag gac aat gac aac aag ctg     960
Leu Gly Thr Leu Leu Ser Tyr Ala Thr Glu Asp Asn Asp Asn Lys Leu
305                 310                 315                 320
gtg ctg cac ggc cga gac tcc ctg ctg ccc gga tcc atc cac ttc gtg     1008
Val Leu His Gly Arg Asp Ser Leu Leu Pro Gly Ser Ile His Phe Val
                325                 330                 335
atc ggg gac ccg gcc ttc agg gag ctg ccc ttg cag ctg ctg ctg gac     1056
Ile Gly Asp Pro Ala Phe Arg Glu Leu Pro Leu Gln Leu Leu Leu Asp
            340                 345                 350
ggc cag tgg cac cac atc tgt gtc atc tgg acg tcc acc cag ggc agg     1104
Gly Gln Trp His His Ile Cys Val Ile Trp Thr Ser Thr Gln Gly Arg
        355                 360                 365
tac tgg ctc cac gtg gat cgc agg ctg gtg gcc acc ggc tcc cgc ttc     1152
Tyr Trp Leu His Val Asp Arg Arg Leu Val Ala Thr Gly Ser Arg Phe
    370                 375                 380
agg gag ggc tat gag atc ccc ccc gga ggg tcc ctc gtg ctg ggc cag     1200
Arg Glu Gly Tyr Glu Ile Pro Pro Gly Gly Ser Leu Val Leu Gly Gln
385                 390                 395                 400
gaa caa gac agc gtg ggg ggc gga ttc gac agc tcc gag gcc ttc gtg     1248
Glu Gln Asp Ser Val Gly Gly Gly Phe Asp Ser Ser Glu Ala Phe Val
                405                 410                 415
ggg agc atg tct ggc ttg gct atc tgg gat cgg gcg ctg gtt ccc ggg     1296
Gly Ser Met Ser Gly Leu Ala Ile Trp Asp Arg Ala Leu Val Pro Gly
            420                 425                 430
gaa gtt gca aac ctt gcc atc ggg aaa gag ttc ccg aca ggt gcc atc     1344
Glu Val Ala Asn Leu Ala Ile Gly Lys Glu Phe Pro Thr Gly Ala Ile
        435                 440                 445
ctg acg ctg gcc aat gct gca cta gca ggc gga ttt gtg cag ggg gcc     1392
Leu Thr Leu Ala Asn Ala Ala Leu Ala Gly Gly Phe Val Gln Gly Ala
    450                 455                 460
aac tgc acc tgc ctg gaa cgc tgt ccc tga                             1422
Asn Cys Thr Cys Leu Glu Arg Cys Pro
465                 470

<210>  41
<211>  473
<212>  PRT
<213>  Homo sapiens

<400>  41

Met Gly Ser Gly Asn Trp Glu Val Thr Gly Pro Pro Cys Gly Ser Arg
1               5                   10                  15
Cys Glu Arg Pro Pro Gly Leu Pro Arg Gly Val His Gly Gln Phe Arg
            20                  25                  30
Ser Ala Val Gly Leu Ser Gly Leu Arg Trp Phe Arg Arg Phe Gln Glu
        35                  40                  45
Val Thr Trp Thr His Leu Gln Asn Ile Ala Ser Asn Tyr Asn Val Ser
    50                  55                  60
Tyr Asn Val Asp Val Arg Phe Arg Ser Leu Ala Glu Glu Ser Gln Ala
65                  70                  75                  80
Val Ala Gln Ala Val Asn Arg Ser Gln Ala Ser Val Gln Gly Glu Leu
                85                  90                  95
Ala Gln Leu Lys Ala Trp Val Arg Lys Leu Gln Arg Arg Gly Arg Lys
            100                 105                 110
Val Asp Thr Arg Leu Arg Ala Leu Asp Leu Thr Leu Gly Glu Arg Ser
        115                 120                 125
Gln Gln Arg Ala Arg Glu Arg Lys Ala His Lys Ala Gln Arg Asp Ala
    130                 135                 140
Leu Gln Asp Ser Leu Ala Arg Leu Glu Gly Leu Val His Ser Gln Gly
145                 150                 155                 160
Ala Arg Leu Ala Ala Leu Glu Gly Arg Leu Pro Val Ala His Pro Gly
                165                 170                 175

Thr Ala Ala Leu Gly Pro Ala Leu Val Pro Thr Pro Thr Gln Pro Glu
                180                 185                 190
Glu Leu Gly Pro Thr Ser Leu Lys Leu Gln Arg Asp Arg Gln Glu Leu
        195                 200                 205
Arg Ala Ala Ser Glu His Arg Gly Pro Pro Gln Asp Ser Ser Ala Pro
    210                 215                 220
Leu Gln Gly Arg Arg Glu Pro Pro Ala Ser Gly Ser His Arg Val Leu
225                 230                 235                 240
Ser Gly Thr Ala Pro Lys Asp Pro Arg Gln Gln Ala Trp Ser Pro Gln
                245                 250                 255
Val Pro Gly Glu Ile Cys Gly Val Gly Pro Thr Leu Val Phe Pro Asn
            260                 265                 270
Ala Ser Thr Arg Asn Val Val Phe Leu Ser Pro Gly Phe Val Thr Ala
        275                 280                 285
Leu Arg Ala Leu Ser Phe Cys Ser Trp Val Arg Thr Ala Ser Gly Arg
    290                 295                 300
Leu Gly Thr Leu Leu Ser Tyr Ala Thr Glu Asp Asn Asp Asn Lys Leu
305                 310                 315                 320
Val Leu His Gly Arg Asp Ser Leu Leu Pro Gly Ser Ile His Phe Val
                325                 330                 335
Ile Gly Asp Pro Ala Phe Arg Glu Leu Pro Leu Gln Leu Leu Leu Asp
            340                 345                 350
Gly Gln Trp His His Ile Cys Val Ile Trp Thr Ser Thr Gln Gly Arg
        355                 360                 365
Tyr Trp Leu His Val Asp Arg Arg Leu Val Ala Thr Gly Ser Arg Phe
    370                 375                 380
Arg Glu Gly Tyr Glu Ile Pro Pro Gly Gly Ser Leu Val Leu Gly Gln
385                 390                 395                 400
Glu Gln Asp Ser Val Gly Gly Gly Phe Asp Ser Ser Glu Ala Phe Val
                405                 410                 415
Gly Ser Met Ser Gly Leu Ala Ile Trp Asp Arg Ala Leu Val Pro Gly
            420                 425                 430
Glu Val Ala Asn Leu Ala Ile Gly Lys Glu Phe Pro Thr Gly Ala Ile
        435                 440                 445
Leu Thr Leu Ala Asn Ala Ala Leu Ala Gly Gly Phe Val Gln Gly Ala
    450                 455                 460
Asn Cys Thr Cys Leu Glu Arg Cys Pro
465                 470

## Description

### Cross-Reference to Related Application

The present patent application is accompanied by a claim of priority based on the Japanese Patent Application No. 2016-097233 (filing date: May 13, 2016) previously filed in Japan. Hereafter, the entire disclosure of the patent applications is incorporated herein by reference.

### BACKGROUND OF INVENTION

### Field of the Invention

The present invention relates to therapeutic agents for obesity-related diseases with inhibitory effects on a hepatic secretory metabolic regulatory factor.

### Background Art

In modern society, obesity and metabolic syndrome complicated by obesity have become one of major problems in the national public health. In this syndrome, it is an essentially important point that risk factors responsible for atherosclerosis form into a cluster, and thus it is urgently needed to elucidate the molecular mechanism of atherosclerosis and to construct a novel therapeutic strategy based on its fundamental findings. In considering of the etiology of metabolic syndrome, it is necessary to understand a mechanism of the onset of insulin resistance, but it has not been fully clarified yet.

The present inventors have proposed that less glucose/carbohydrate assimilation than lipid assimilation occurred in energy metabolism of insulin resistance. In other words, a disturbed balance of carbohydrate/lipid metabolism (carbohydrate-lipid imbalance) was essential to the onset of insulin resistance (non-patent document 1). An integrated metabolic regulatory mechanism which cannot be explained from an individual aspect in metabolic profile of each organ will be clarified by elucidating the regulatory mechanism of carbohydrate/lipid metabolism (carbohydrate-lipid balance). From this point of view, the present inventors showed that metabolic information induced physiological lipolysis via the hepatic vagal nerve in response to glycogen consumption in the liver at starvation (non-patent document 2). The present inventors revealed the presence of a novel "glycogen sensor" which was able to detect glycogen consumption as the basis for intrahepatic metabolism underlying these regulatory mechanisms (non-patent document 2). Furthermore, the present inventors also revealed that neuronal circuits that regulated metabolism transmitted neuronal signals to the central nervous system via the hepatic vagal nerve, from which adipose tissues were stimulated through the efferent sympathetic nerves, resulting in lipolysis (non-patent document 2).

However, it had already been reported for the first time in Japan by Katagiri et al. at Tohoku University in 2006 that there was neuronal circuit itself involved in metabolic regulation, and that it had been attracting attention from the world (non-patent document 3). Nevertheless, how this mechanism converts metabolism-related information from the liver into neural information still remained unresolved.

### PRIOR ART DOCUMENTS

### NON-PATENT DOCUMENTS

[Non-patent document 1] Ide T. Nature Cell Biol. 2004 Apr, 6(4): 351-7
[Non-patent document 2] Izumida Y. Nature Commun 4: 2316, 2013
[Non-patent document 3] Uno K, Katagiri H, et al., Science. 16. 312(5780): 1659-9, 2006

### SUMMARY OF THE INVENTION

The present inventors dedicated their efforts to elucidate a mechanism underlying for controlling energy homeostasis mediated by neural metabolic regulation through the investigation of a mechanism that can translate hepatic metabolic patterns into common metabolic information including neurogenic information. Consequently, the present inventors found that the inhibition of pentraxin-4 synthesized in the liver facilitated burning of triglycerides as hepatic intracellular energy sources more predominantly than carbohydrates stored as glycogen, as well as decrease in fat mass by promoting lipolysis in adipose tissues, and conversion of the adipose tissues into brown adipose tissues. In total, these mechanisms leaded to enhanced β-oxidation, suppressed inflammation and reduced oxidative stress in the liver. The present invention is based on these findings.

Therefore, according to the present invention, methods of drug screening and pharmaceutical compositions for treatment of obesity-related diseases would be provided.

The present invention includes the followings:
(1) A method for screening a drug for treating obesity-related diseases characterized by using an ability of a candidate drug to inhibit the action of pentraxin-4 as an index, and by identifying a candidate drug which has the ability as a drug for treating obesity-related diseases.
(2) The method according to (1), wherein the index is an ability to inhibit the expression of pentraxin-4 gene.
(3) The method according to (2), wherein the index is an ability to inhibit the initiation of transcription from pentraxin-4 gene to mRNA, an ability to degrade mRNA from pentraxin-4 gene, or an ability to inhibit translation from the pentraxin-4 mRNA to protein.
(4) The method according to (2) or (3), characterized by using a nucleic acid molecule in which a DNA fragment comprising the transcriptional regulatory region of pentraxin-4 gene and a DNA fragment comprising a reporter gene are operably ligated.
(5) The method according to (1), wherein the index is an ability to inhibit the function of pentraxin-4 protein.
(6) The method according to (5), wherein the index is an ability to inhibit the extracellular secretion of the pentraxin-4 protein, an ability to inhibit the transfer of the pentraxin-4 protein into neurons or an ability to inhibit signal transduction from the pentraxin-4 protein.
(7) The method according to (5) or (6), comprising a step of selecting a substance which specifically binds to pentraxin-4 protein as a primary candidate.
(8) The method according to any one of (1) to (7), wherein a drug to be screened is a drug for improvement of obesity due to enhanced lipolysis, improvement of insulin resistance, anti-obesity due to beige adipogenesis, decreased liver fat accumulation, decreased hepatic inflammation, anti-oxidative stress, or hepatic anti-fibrotic response.
(9) The method according to any one of (1) to (8), wherein the obesity-related disease is non-alcoholic steatohepatitis (NASH) or diabetes mellitus.
(10) A pharmaceutical composition comprising a pentraxin-4 inhibitor, for treating obesity-related diseases.
(11) The pharmaceutical composition according to (10), wherein the pentraxin-4 inhibitor is an RNAi mediator which specifically inhibits the expression of pentraxin-4 gene.
(12) The pharmaceutical composition according to (10), wherein the pentraxin-4 inhibitor is a vector expressing an RNAi mediator which specifically inhibits the expression of pentraxin-4 gene.
(13) The pharmaceutical composition according to (10), wherein the pentraxin-4 inhibitor is an antibody which specifically binds to pentraxin-4 protein.
(14) The pharmaceutical composition according to (10), wherein the pentraxin-4 inhibitor is an antibody which recognizes a peptide of amino acid sequence as set forth in SEQ ID NO 4.
(15) The pharmaceutical composition according to (13) or (14), wherein the antibody is monoclonal antibody.
(16) The pharmaceutical composition according to any one of (10) to (15), for improvement of obesity due to enhanced lipolysis, improvement of insulin resistance, anti-obesity due to beige adipogenesis, decreased liver fat accumulation, decreased hepatic inflammation, anti-oxidative stress, and hepatic anti-fibrotic response.
(17) The pharmaceutical composition according to any one of (10) to (16), wherein the obesity-related disease is non-alcoholic steatohepatitis (NASH) or diabetes mellitus.
(18) A pentraxin-4 inhibitor for use in therapy.
(19) A pentraxin-4 inhibitor for use in the treatment of obesity-related diseases.
(20) A use of a pentraxin-4 inhibitor for the manufacture of a medicament for the treatment of obesity-related diseases.
(21) A method for treating obesity-related diseases comprising administering a therapeutically effective dose of pentraxin-4 inhibitor to a subject in need thereof.

According to the present invention, it is possible to optimize the regulation of energy metabolism by intentionally modifying a regulatory mechanism of carbohydrate/lipid metabolism by neural regulation. For example, according to the present invention, it is possible to improve obesity due to the acceleration of lipolysis, to improve insulin resistance, or to enhance basal metabolism due to thermogenesis. Additionally, according to the present invention, it is possible to get several effects such as anti-obesity due to beige adipogenesis, decreased liver fat accumulation, deceased hepatic inflammation, or anti-oxidative stress or hepatic anti-fibrotic response. Furthermore, according to the present invention, it is possible to provide novel therapeutic agents for obesity-related diseases such as nonalcoholic steatohepatitis (NASH) or diabetes mellitus. Thus, according to the present invention, optimizing the regulation of energy metabolism, intentionally modifying the regulatory mechanism of carbohydrate/lipid metabolism controlled by neuronal circuits, can provide novel therapeutic agents for obesity-related diseases such as NASH occurred due to excessive neutral fat accumulation underlying obesity and diabetes mellitus.

### BRIEF DESCRIPTION OF DRAWINGS

Figure 1 shows an appearance of the suspension of liver tissue homogenate in wild-type mice. Floating substances in the supernatant (indicated by open arrowheads) are lipid components, and pellets (indicated by shaded arrowheads) are glycated substances such as glycogen.
Figure 2 shows the contents of glycogen and triglyceride in the liver tissues of wild-type mice. Open bars indicate control (LacZ-i) group, and closed bars indicate Ptx4-inhibition (Ptx4-i) group.
Figure 3 shows an appearance of epididymal adipose tissues in wild-type mice. The top row indicates a specimen, showing white tone of color, obtained from control (LacZ-i) group. The bottom row indicates a specimen showing reddish-brown obtained from Ptx4-inhibition (Ptx4-i) group. The scale bar is 5mm.
Figure 4 shows the body weight of wild-type mice and the weights of epididymal adipose tissues and brown adipose tissues. Open bars indicate control (LacZ-i) group, and closed bars indicate Ptx4-inhibition (Ptx4-i) group. The Y-axis in the panel showing the result of the weight of epididymal adipose tissues represents the weight percentage of epididymal adipose tissues as WAT (%). The Y-axis in the panel showing the result of the weight of brown adipose tissues represents the weight percentage of brown adipose tissues as BAT (%).
Figure 5 shows mRNA levels expressed in white adipose tissues of high-fat diet induced obesity mice. Open bars indicate control (LacZ-i) group, and closed bars indicate Ptx4-inhibition (Ptx4-i) group.
Figure 6 shows the expression levels of mRNA in epididymal adipose tissues of non-alcoholic steatohepatitis model mice. Open bars indicate control (LacZ-i) group, and closed bars indicate Ptx4-inhibition (Ptx4-i) group.
Figure 7 shows mRNA expression levels of the genes associated with carbohydrate and lipid metabolism, inflammation-associated genes, and β-oxidation-associated gene in the liver of wild-type mice. Open bars indicate control (LacZ-i) group, and closed bars indicate Ptx4-inhibition (Ptx4-i) group. The Y-axes represent the ratios of expression levels in Ptx4-inhibition group to those of control group.
Figure 8 shows mRNA levels expressed in the liver of wild-type mice. Open bar indicates control (LacZ-i) group, and closed bar indicates Ptx4-inhibition (Ptx4-i) group.
Figure 9 shows plasma glucose and lipid contents in wild-type mice. Open bars indicate control (LacZ-i) group, and closed bars indicate Ptx4-inhibition (Ptx4-i) group.
Figure 10 shows the expression levels of insulin signaling-associated proteins in the liver of wild-type mice by Western blotting. Left: control (LacZ-i) group. Right: Ptx4-inhibition (Ptx4-i) group.
Figure 11 shows pictures of the liver tissues stained with periodic acid-schiff (PAS) in leptin gene-deficient mice. Left: control (LacZ-i) group. Right: Ptx4-inhibition (Ptx4-i) group. Upper panels: 40x magnification. Lower panels: 200x magnification. Asterisk indicates a central vein of the liver, and arrowheads point out lipid droplets.
Figure 12 shows the contents of glycogen and triglyceride in the liver tissues of leptin gene-deficient mice. Open bars indicate control (LacZ-i) group, and closed bars indicate Ptx4-inhibition (Ptx4-i) group.
Figure 13 shows mRNA expression levels of carbohydrate/lipid metabolism-associated genes, inflammation- associated genes, and β-oxidation-associated gene in the liver of leptin gene-deficient mice. Open bars indicate control (LacZ-i) group, and closed bars indicate Ptx4-inhibition (Ptx4-i) group. The Y-axes represent the ratios of expression levels in Ptx4-inhibition group to those of control group.
Figure 14 shows mRNA expression levels of oxidative stress related genes in the liver of leptin gene-deficient mice. Open bar indicates control (LacZ-i) group, and closed bar indicates Ptx4-inhibition (Ptx4-i) group. The Y-axis shows the ratio of Sod2 gene expression level to 36b4 gene expression level.
Figure 15 shows mRNA expression levels of lipid metabolism-associated genes, inflammation-associated genes, β-oxidation-associated gene, and oxidative stress related gene in the liver of high-fat diet induced obesity mice. Open bars indicate control (LacZ-i) group, and closed bars indicate Ptx4-inhibition (Ptx4-i) group. In the panel showing the results of oxidative stress-related gene, the Y-axis means the ratio of Sod2 gene expression level to 36b4 gene expression level.
Figure 16 shows blood glucose levels and plasma insulin concentrations in high-fat diet induced obesity mice. Open bars indicate control (LacZ-i) group, and closed bars indicate Ptx4-inhibition (Ptx4-i) group.
Figure 17 shows the contents of glycogen and triglyceride in the liver tissues of non-alcoholic steatohepatitis model mice. Open bars indicate control (LacZ-i) group, and closed bars indicate Ptx4-inhibition (Ptx4-i) group.
Figure 18 shows pictures of the liver tissues stained with hematoxylin-eosin (HE) in non-alcoholic steatohepatitis model mice. Left: control (LacZ-i) group. Right: Ptx4-inhibition (Ptx4-i) group. In Ptx4-inhibition (Ptx4-i) group, decrease in the areas of void regions around the portal vein (PV), suggesting of decreased lipid droplets, is noted.

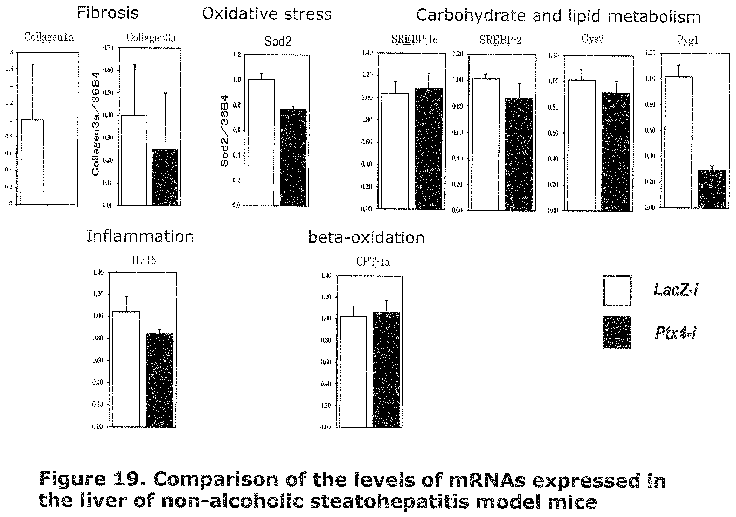

Figure 19 shows mRNA expression levels of fibrosis-related genes, oxidative stress-related genes, carbohydrate/lipid metabolism-associated genes, inflammation-related gene, and β-oxidation-associated gene in the liver of non-alcoholic steatohepatitis model mice. Open bars indicate control (LacZ-i) group, and closed bars indicate Ptx4-inhibition (Ptx4-i) group. In the panel showing the result of oxidative stress-related gene, the Y-axis means the ratio of Sod2 gene expression level to 36b4 gene expression level. Similarly, in the panel showing the result of fibrosis-related genes (collagen3a), the Y-axis means the ratio of collagen3a gene expression levels to 36b4 gene expression level.

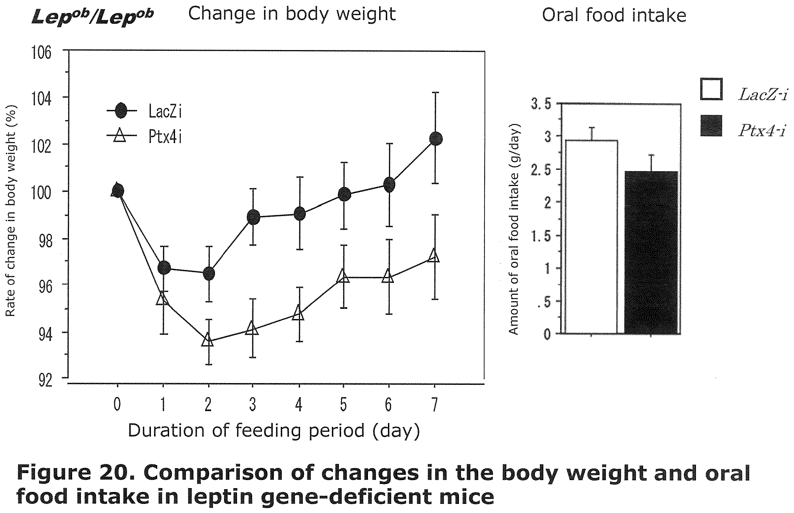

Figure 20 shows changes in the body weight and oral food intake in leptin gene-deficient mice. In the panel showing the result of changes in the body weight, the X-axis indicates the duration of feeding periods after mice received Ptx4i adenoviral vector (pAd-Ptx4i) (when mice received the vector on day 0), and the Y-axis indicates the change in the body weight (when the body weight on day 0 was defined as 100%). Closed circles indicate control (LacZ-i) group, and open triangles indicate Ptx4-inhibition (Ptx4-i) group. In the panel showing the result of oral food intake, the Y-axis indicates oral food intake (g) for 1 day per mouse 7 days after the administration of pAd-Ptx4i. Open bar indicates control (LacZ-i) group, and closed bar indicates Ptx4-inhibition (Ptx4-i) group.
Figure 21 shows analysis of respiratory metabolism in wild-type mice. Open bars indicate control (LacZ-i) group, and closed bars indicate Ptx4-inhibition (Ptx4-i) group.
Figure 22 shows rectal temperatures and UCP2 mRNA expression levels of the liver in leptin gene-deficient mice. Open bars indicate control (LacZ-i) group, and closed bars indicate Ptx4-inhibition (Ptx4-i) group. In the panel showing the result of UCP2 mRNA expression levels, the Y-axis represents the ratio of expression levels in Ptx4-inhibition group to that of control group.
Figure 23 shows cumulative food intake in wild-type mice. Open circles represent control group (receiving rabbit IgG antibody), and closed circles represent the experimental group receiving anti-rabbit IgG polyclonal antibody.
Figure 24 shows the weights of epididymal adipose tissues in wild-type mice. Open bars represent control group (receiving rabbit IgG antibody), and closed bars represent the experimental group receiving anti- rabbit IgG polyclonal antibody.

### DETAILED DESCRIPTION OF THE INVENTION

The present inventors identified pentraxin-4 gene defining a molecule of which hepatic secretion was regulated in correlation with hepatic glycogen consumption, and found that this newly identified hepatic secretory metabolic regulatory factor was involved in signal transduction of intracellular or extracellular signaling, as well as not only metabolic regulation in hepatocytes but also lipolysis and brown adipogenesis in adipose tissues. In glycogen-sensing mechanism induced by this newly identified hepatic secretory metabolic regulatory factor, actively regulates lipolysis, energy consumption, and feeding behavior are strongly regulated. Thus, it is possible to treat obesity-related diseases by the functional inhibition of pentraxin-4.

Pentraxin-4 is a member of pentraxin superfamily, multifunctional proteins characterized by having pentraxin-specific domain containing the consensus sequence consisting of 8 amino acids conserved on its C-terminal. The information of human pentraxin-4 gene including its genomic sequence, mRNA sequence and amino acid sequence based on the corresponding mRNA is registered as Gene ID: 390667 in the NCBI database. The sequence of mRNA (cDNA) registered in this database is shown in SEQ ID NO 26, and the amino acid sequence is also shown in SEQ ID NO 27. In the present description, pentraxin-4 gene and pentraxin-4 protein are sometimes abbreviated as "Ptx4 gene" and "Ptx4 protein", respectively.

In the present invention, obesity-related diseases include diseases such as nonalcoholic steatohepatitis (NASH) and diabetes mellitus, in which therapeutic effects can be expected by preferentially burning triglycerides as compared to glycogens as carbohydrates. Additionally, the treatment of obesity-related diseases in the present invention includes not only the radical treatment of diseases but also the improvement of their symptoms. For example, the improvement includes the improvement of obesity due to enhanced lipolysis, improvement of insulin resistance, and increased basal metabolism due to increased thermogenesis. Furthermore, according to another embodiment, the treatment of obesity-related diseases in the present invention includes the improvement of obesity due to enhanced lipolysis, improvement of insulin resistance, anti-obesity due to beige adipogenesis, decreased liver fat accumulation, decreased hepatic inflammation, anti-oxidative stress, and hepatic anti-fibrotic response.

According to the present invention, methods for drug screening of obesity-related diseases can be provided. In these methods, the ability of a potential drug candidate to inhibit the action of pentraxin-4 is used as an index, so the candidate having this ability is identified as a pharmaceutical drug for obesity-related diseases. Here, the inhibition of pentraxin-4 action may be either the inhibition of pentraxin-4 gene expression or the inhibition of pentraxin-4 protein function.

According to one embodiment of the present invention, the pharmaceutical drug mentioned above is screened using its ability to inhibit pentraxin-4 gene expression as an index. Such abilities include an ability to inhibit the initiation of transcription from pentraxin-4 gene to mRNA, to degrade mRNA from pentraxin-4 gene, and to inhibit of translation from the pentraxin-4 mRNA to its corresponding protein.

According to another embodiment of the present invention, the pharmaceutical drug mentioned above is screened using its ability to inhibit the function of pentraxin-4 protein as an index. Such abilities include an ability to inhibit extracellular secretion of pentraxin-4 protein, to inhibit the transfer of it into neurons, and to inhibit signal transduction.

The screening methods of the present invention can be adequately performed by those skilled in the art depending on indices used for drug screening. After preparing cells expressing pentraxin-4 genes under the same conditions as in human body, for example, hepatocytes or genetically modified cells that were able to express pentraxin-4 gene induced by gene transfer, an expression assay for pentraxin-4 gene under the presence or absence of candidate compounds is performed using the prepared cells. Subsequently, the inhibitory potency of them on pentraxin-4 gene expression can be measured by comparison in the amount of pentraxin-4 mRNA or of pentraxin-4 protein. Similarly, after preparing an assay system in which pentraxin-4 protein acts under the same conditions as in human body, the ability of candidate compounds to inhibit the function of pentraxin-4 protein (for extracellular secretion, transfer into neurons, and signal transduction) can be measured by performing a test under the presence or absence of candidate compounds, and comparing abilities of pentraxin-4 protein. According to an embodiment of the present invention, after lipopolysaccharide (LPS) is administered to cells derived from stromal cell line, intracellular calcium concentrations, derived from influx occurred when pentraxin-4 transcription is promoted (Martinez de la Torre Y et al., J. Immunol. 184 (9), 5055-5064. 2010) and/or extracellular secretion of pentraxin-4 is evoked, are measured as changes in fluorescent intensities using fluorescent calcium indicators. By using this assay system, drugs for obesity-related diseases can be identified and screened by selecting candidate compounds having an inhibitory effect on pentraxin-4.

When drugs for obesity-related diseases are screened using an ability to inhibit pentraxin-4 gene expression as an index, it is desirable to use nucleic acid molecules in which a DNA fragment comprising transcriptional regulatory region of pentraxin-4 gene and a DNA fragment comprising a reporter gene are operably ligated. Here, the base sequence of transcriptional regulatory regions of human pentraxin-4 gene can be obtained from the information of Gene ID: 390667 registered in the NCBI database. Additionally, the base sequence of transcriptional regulatory regions of mouse pentraxin-4 gene can be obtained from the information of Gene ID: 68509 registered in the NCBI database. Furthermore, any reporter gene system can be used if the method for quantitative detection of the gene products has been established. For example, the following genes are available: genes encoding bioluminescent proteins such as luciferase, encoding enzymes such as β-galactosidase and chloramphenicol acetyltransferase (CAT), and encoding fluorescent proteins such as humanized Azami green (HmAG) fluorescent protein, green fluorescent protein (GFP) and red fluorescent protein (DS-RED). Among them, genes encoding bioluminescent proteins are more preferably used, and the gene encoding luciferase is much more preferably used. Furthermore, "operably ligated" means that genes linked to upstream (5' sides) or downstream (3' sides) of transcriptional regulatory regions are connected to have an enhanced or reduced transcriptional activity by the transcriptional regulatory region. When the ability of candidate compounds to inhibit pentraxin-4 gene expression is detected or evaluated, in vitro assay system for transcription activity in which the nucleic acid molecules mentioned above were introduced or cells transformed by the nucleic acid molecules can be used. Such cells for this assay include mammalian cells, insect cells, yeasts, and Escherichia coli. However, it is more preferable to use mammalian cells. The ability to inhibit pentraxin-4 gene expression can be detected or evaluated based on the extent of reporter gene expression when candidate compounds were administered to an in vitro assay system for transcriptional activity mentioned above or were mixed with transformed cells mentioned above. For general methods relevant to the above such as genetic engineering techniques, detection of reporter gene expression and screening of candidate compounds, well-known techniques for those skilled in the arts can be used.

When candidate compounds are screened based on their ability to inhibit the function of pentraxin-4 protein as an index, it is desirable to have a step to select substances that specifically bind to pentraxin-4 protein as primary candidates. Herein, more preferably, pentraxin-4 protein is immobilized. In the step for selection, any technique generally known to those skilled in the art can be used. For example, when test substances are cyclic peptides, the selection can be performed according to TRAP display (T. Ishizawa, T. Kawakami, P. C. Reid, H. Murakami (2013) J. Am. Chem. Soc. 135, 5433-5440) or the methods described in the Japanese Unexamined Patent Application Publication No. 2014-127,352, the Japanese Unexamined Patent Application Publication No. 2013-046637, WO2014/119600, or WO2011/049157. Primary candidate substances obtained by such selection methods above can be further screened by subjecting them to an assay system for obesity-related diseases described in the present description.

In the screening methods of the present invention, various types of substances such as small molecules, low-molecular compounds, high-molecular compounds, nucleic acid molecules, proteins, and peptides (including cyclic peptides) can be used as candidate compounds.

According to the present invention, pharmaceutical compositions, comprising pentraxin-4 inhibitor, for treating obesity-related diseases can be provided. Furthermore, according to the present invention, methods for treating or preventing obesity-related diseases, comprising the administration of therapeutically effective dose of pentraxin-4 inhibitor to subjects who require medical treatment, can be provided. Moreover, according to the present invention, use of pentraxin-4 inhibitor for the manufacture of a medicament for treating obesity-related diseases can be provided. Additionally, according to the present invention, pentraxin-4 inhibitors for treating obesity-related diseases can be provided. As pentraxin-4 inhibitors, various types of substances such as small molecules, low-molecular compounds, high molecular compounds, nucleic acid molecules, proteins, and peptides (including cyclic peptides) can be used.

Subjects who receive treatment or prophylaxis are preferably mammals, for example, human or non-human mammals.

According to a preferable embodiment of the present invention, pentraxin-4 inhibitors are RNAi mediators that specifically inhibit pentraxin-4 gene expression. In the present description, "RNAi mediators" mean not only siRNA itself but also double-stranded RNA molecules that is longer and can transfer siRNA into targeted cells. RNAi mediators are well-known tool that can inhibit specific gene expression via RNA interference (RNAi) (Elbashir, S.M. et al., Nature 411, 494-498, 2001). Typically, siRNA comprises nucleotide sequences of 19 to 21 base pairs that are homologous to sequences that are specific to mRNAs of target genes. The double-stranded RNA molecule mentioned above typically comprise a longer nucleotide sequence having a moiety corresponding to siRNA. The double-stranded RNA molecules may have a hairpin structure. The RNAi mediators can be easily designed by those skilled in the art based on sequences of genes of which expressions are to be inhibited.

Furthermore, the RNAi mediators mentioned above can be expressed by an appropriate vector that was delivered into cells. Accordingly, pentraxin-4 inhibitors may be vectors expressing RNAi mediators that specifically inhibit pentraxin-4 gene expression. Such vectors can be easily constructed by well-known standard procedures in the art (Bass, B.L., Cell 101, 235-238, 2000, Tavernarakis, N. et al., Nat. Genet. 24, 180-183, 2000; Malagon, F. et al., Mol. Gen. Genet. 259, 639-644, 1998; Parrish, S. et al., Mol. Cell 6, 1077-1087, 2000).

The following Table 1 shows an example of base sequence of RNAi mediator having an inhibitory effect on pentraxin-4, used in the present invention (RNAi base sequence 1).

### [Table 1]

**Table 1. An example of nucleotide sequence of RNAi mediator having an inhibitory effect on the function of pentraxin-4 gene (RNAi base sequence 1)**

| SEQ ID NO | Nucleotide sequence¹⁾ | Remarks |
|---|---|---|
| 1 | 5'-TGAtAAtAAGTTAGTACTa-ACGTGTGCTGTCCGT-CAGTACTAACTTGTTGTCA-3' | Sense strand |
| 2 | 3'-TGACAACAAGTTAGTACTG-ACGGACAGCACACGT-tAGTACTAACTTaTTaTCAC-5' | Antisense strand |

| | | |
|---|---|---|
| 1) All small letters of alphabet indicate mismatched bases that are intentionally inserted | | |

In addition to the base sequences as shown in "RNAi base sequence 1" mentioned above, base sequences may have mutations including insertion, deletion and substitution of nucleotide residues as long as possessing an inhibitory effect on pentraxin-4 gene expression. The position of such mutations is not particularly limited. Also, the number of such mutations is not particularly limited, but is, for example, one-to-few, preferably 1 to 5, more preferably 1 to 3, further more preferably 1 or 2, and most preferably 1. This mutation may be any one of insertion, deletion and substitution or a combination thereof, but is preferably substitution.

According to another preferable embodiment of the present invention, pentraxin-4 inhibitors are antibodies that recognize whole or partial peptide of pentraxin-4 protein. More preferably, pentraxin-4 inhibitors are antibodies that specifically bind to pentraxin-4 protein. Antibodies may be either polyclonal or monoclonal antibodies, but preferably are monoclonal antibodies. Furthermore, it is preferable that antibodies not only specifically bind to pentraxin-4 protein but also can inhibit the function of pentraxin-4 protein.

Examples of amino acid sequences of antigenic peptides for producing antibodies used in the present invention are shown in the following Table 2 (amino acid sequences 1 to 3). Amino acid sequence 1 and Amino acid sequence 2 contain amino acid residues of 221 to 234 and of 406 to 418 of mouse pentraxin-4 protein, respectively. Amino acid sequence 3 contains amino acid residues of human pentraxin-4 protein that is corresponding to amino acid residues of 406 to 418 of mouse pentraxin-4 protein. Amino acid sequence 2 and Amino acid sequence 3 are highly homologous, and an antibody produced using antigenic peptides corresponding to Amino acid sequence 2 is more likely to have cross-immunoreactivity to human pentraxin-4 protein.

### [Table 2]

**Table 2. Examples of amino acid sequences of antigenic peptides for producing antibodies that specifically bind to pentraxin-4 protein.**

| | SEQ ID NO | Amino acid sequence¹⁾ |
|---|---|---|
| Amino acid sequence 1 | 3 | NH₂-C+QVAKSSPQHRSSPH-COOH |
| Amino acid sequence 2 | 4 | NH₂-C+EQDTVGGEFDSSE-COOH |
| Amino acid sequence 3 | 5 | NH₂-C+EQDSVGGGFDSSE-COOH |

| | | |
|---|---|---|
| 1) Amino acid sequences of antigenic peptides used when producing antibodies that specifically bind to pentraxin-4 protein are indicated. These sequences contain cysteine residues that were intentionally added to the N-terminal. | | |

In addition to Amino acid sequences 1 to 3 described above, amino acid sequences may have mutations including insertion, deletion, or substitution of amino acid residue(s) as long as an antibody has an inhibitory potency on pentraxin-4. The position of such mutation is not particularly limited. Also, the number of such mutations is not particularly limited, but, for example, one-to-few, preferably 1 to 5, more preferably 1 to 3, further more preferably 1 or 2, and most preferably 1. These mutations may be any one of insertion, deletion and substitution or a combination thereof, but is preferably substitution, and more preferably conservative substitution (substituting a specific amino acid to different amino acid having similar property).

According to another preferable embodiment of the present invention, pentraxin-4 inhibitors are small molecules. Such small molecules can be selected by screening methods of the present invention.

Pentraxin-4 inhibitors can be administered by an appropriate route such as topical, intravenous, subcutaneous, intramuscular, oral, rectal, and mucosal routes. Physicians or veterinarians can adequately determine the therapeutically effective dose of pentraxin-4 inhibitor depending on the severity of symptoms, age of subjects, and the effectiveness, administration route, and frequency of administration, and the like of each drug to be used. In general, a therapeutically effective dose is approximately 0.001 to 1000 mg/body weight (kg) per day, and preferably approximately 0.01 to 10 mg/body weight (kg) per day, more preferably approximately 0.01 to 1 mg/body weight (kg) per day.

A pentraxin-4 inhibitor can be administered in combination with drugs other than pentraxin-4 inhibitor for obesity-related diseases. Herein, "administered in combination with" may be either of the following: (i) to administer as the mixture of pentraxin-4 inhibitor and other drug(s) in one formulation, (ii) to prepare separately pentraxin-4 inhibitor and other drug(s) and to administer them at the same timing, (iii) to prepare separately pentraxin-4 inhibitor and other drug(s) and to administer them at the different timing. Dosage and administration, timing of administration, and administration intervals of pentraxin-4 inhibitor and other drug(s) can be determined by general technical knowledge commonly known to those skilled in the art.

A pentraxin-4 inhibitor can be administered in combination with a pharmaceutically acceptable carrier. Thus, according to the present invention, provided is a pharmaceutical composition for treating obesity-related diseases comprising a pentraxin-4 inhibitor and a pharmaceutically acceptable carrier thereof.

Pharmaceutically acceptable carriers such as vehicles, excipients, and diluents can be appropriately selected by those who skilled in the art depending on administration routes and the property of each drug to be used.

### EXAMPLES

The present invention will be explained explicitly by way of the following examples, but the present invention is not limited to these examples.

### Example A: Methods

### Example A (1): Animals

Male C57BL/6J mice (7-week-old) were purchased from Japan CREA, Sankyo Laboratory. Leptin gene-deficient mice (Lep^{ob}/Lep^{ob}) were obtained from Japan CREA. Mice were housed in cage and placed under 12-hour light-dark cycle at room temperature (24°C) unless otherwise a different temperature is later specified.

In all the experiments except for the purpose of producing high-fat diet induced obesity (Diet-induced obesity (DIO)) mice and nonalcoholic steatohepatitis (NASH) model mice, standard feed was used which contains 25.6% (wt/wt) protein, 3.8% fiber, 6.9% ash constituent, 50.5% carbohydrate, 4% fat and 9.2% of moisture content. Male C57BL/6J mice (7-week-old) were fed with high-fat diet (60% fat contents by use of lard) in order to produce high-fat diet induced obesity (Diet-induced obesity (DIO)) mice. Additionally, male C57BL/6J mice (7-week-old) were also fed with choline-deficient, methionine-defined, high-fat diet (CDAHFD, A06071302) provided from Research Diet Corporation for three weeks to produce nonalcoholic steatohepatitis (NASH) model mice.

### Example A (2): Production of Ptx4 RNAi adenoviral vector

In order to construct a vector expressing RNAi mediator that specifically inhibits pentraxin-4 gene expression, the oligomer for HEK293 cells given in "RNAi base sequence 1" (the sequence is listed in Table 1) is inserted into an entry vector pENTR/U6, and the recombinant vector is mixed with an adenoviral vector pAd-DEST and Gateway LR Clonase (Invitrogen), and the mixture was introduced into DH5a Escherichia coli strain, to obtain adenoviral recombinant DNA (shPtx4 adenoviral vector; pAd-Ptx4i). The obtained pAd-Ptx4i is transfected and amplified into HEK293 cells. Subsequently, the vector was concentrated and purified up to 1 to 5x10¹¹ plaque-forming units (p.f.u.)/ml using PD-10 column (BD healthcare).

### Example A (3): Transfection of Ptx4 RNAi adenoviral vector into mice and preparation of each tissue specimen

Ptx4i adenoviral vector (pAd-Ptx4i) was intravenously administered into mice at the dose of 2 to 8x10⁸ plaque-forming units (p.f.u.)/body. The epididymal adipose tissues and the liver were dissected from mice at each time point and were quickly frozen in liquid nitrogen. Aliquots of dissected tissues were fixed with 4% paraformaldehyde (PFA) as histological specimens.

### Example B: Analysis

### Example B (1): Analysis of liver fat contents

After the liver tissues (each 0.2 g) stored in liquid nitrogen were homogenized using crushing equipment, each homogenate was weighed in a microtube. Each 200 mg of aliquot was taken from the homogenate. After 2 ml of chloroform/methanol (2:1, v/v) was added to each aliquot, intrahepatic lipid was extracted into an organic solvent at 4°C for 48 hours. Subsequently, 100 µl of the obtained extract was injected into a glass tube, and the extract was dried, and the organic solvent in an organic solvent extraction solution was naturally dried and later removed. The pellet of extract was dissolved in isopropanol, and its lipid content was measured by enzyme assay (Wako TG measurement kit). The absorbance of the reaction solution was measured, and lipid amount (mg) per 1 g of the liver tissue was calculated.

### Example B (2): Analysis of hepatic glycogen contents

The liver tissue was homogenized in 5 volumes (v/w, liver weight) of 0.6 N perchloric acid using crushing equipment. To the homogenate (0.1 g), was added 250 µl of 30% potassium hydroxide solution, and the mixture was boiled for 5 minutes under stirring and then cooled it on ice. Subsequently, 50 µl of 2% sodium sulfate and 800 µl of 66% ethanol were added to the mixture, and then the mixture was stirred. The pellet was rewashed in 66% ethanol and suspended in 3 ml of distilled water. After amyloglucosidase solution was added to the suspension, the suspension was incubated under stirring in a warm bath at 55°C for 30 minutes. Glycogen content in the obtained supernatant was measured at the absorbance of 570 nm by glucose oxidase (GOD) assay. Glycogen amount (mg) per 1 g of the liver tissue was calculated from the absorbance.

### Example B (3): Plasma biochemical analysis

In a plasma analysis, the amount of insulin in mice plasma was measured using Ultrasensitive Mouse Insulin Measurement Kit (Morinaga). The amounts of triglyceride, glucose and free fatty acids in mice plasma were measured by using Triglyceride Test Wako, Glucose CII Test Wako, and NEFA C-Test Wako (Wako Pure Chemicals), respectively.

### Example B (4): Analysis of gene expression levels in the tissues Measurement of RNA expression levels

One slice of the liver tissue was placed in 1.5 ml of TRIzol (Invitrogen), and the mixture was immediately homogenized using crushing equipment. The homogenate was centrifuged at 15000 rpm for 5 minutes to collect a supernatant. The supernatant, was added to 0.3 ml of chloroform. After stirring, the mixture was centrifuged again at 15000 rpm for 15 minutes to collect the upper layer of the solution. To the upper layer obtained was added 0.75 ml of isopropanol, and the mixture was centrifuged at 15000 rpm for 15minutes to precipitate mRNA. The pellet containing mRNA was washed in 80% ethanol twice, dissolved in 0.5 ml of distilled water, and was used for the measurement of mRNA amount. After adjusting mRNA to the same amount, cDNA was produced using reverse transcriptase (High Capacity cDNA Reverse Transcription Kit; Applied Biosystems). Gene expression levels were measured using the cDNA obtained and a primer of target genes prepared by quantitative RT-PCR (SYBR Green Dye; Roche Applied Science). The target genes and primer designs used for the measurement of gene expression levels are shown in the following (Tables 3 and 4).

### [Table 3]

**Table 3. Target genes and primer designs used for the quantification of gene expression levels. (No.1)**

| Name of target gene | SEQ ID NO | Type | Nucleotide sequence |
|---|---|---|---|
| Ptx4 | 6 | Forward | GTCACTGTCCACGTTCAGG |
| | 7 | Reverse | GTCCTTGGTAGGTCCTTGGT |
| Gys2 | 8 | Forward | GGAAGAAACTCTATGACGGGTTATT |
| | 9 | Reverse | CATCGATCATATTGTGAGTGGTC |
| Cpt-1a | 10 | Forward | GGACATTATCACCTTGTTTGGC |
| | 11 | Reverse | GGAGCAACACCTATTCATTTGG |
| UCP-2 | 12 | Forward | GACCTCATCAAAGATACTCTCCTGAA |
| | 13 | Reverse | ATCTCGTCTTGACCACATCAACAG |
| UCP-1 | 14 | Forward | AGGATGGTGAACCCGACAAC |
| | 15 | Reverse | TTGGATCTGAAGGCGGACTT |
| IL-1β | 16 | Forward | AACCTGCTGGTGTGTGACGTTC |
| | 17 | Reverse | CAGCACGAGGCTTTTTTGTTGT |
| RelA (NF-kB) | 18 | Forward | TGTGGAGATCATCGAACAGCCG |
| | 19 | Reverse | TTCCTGGTCCTGTGTAGCCATTGAT |
| IKK-β (Ikbkb) | 20 | Forwa rd | TTCCAGCTGAGGAAAGTGTG |
| | 21 | Reverse | AGCTGTTATTCCGGAGGAGA |
| SREBP-1c | 22 | Forward | CGGCGCGGAAGCTGT |
| | 23 | Reverse | TGCAATCCATGGCTCCGT |
| SREBP2 | 24 | Forward | CTGCAGCCTCAAGTGCAAAG |
| | 25 | Reverse | CAGTGTGCCATTGGCTGTCT |

### [Table 4]

**Table 4. Target genes and primer designs used for the quantification of gene expression levels. (No.2)**

| Name of target gene | SEQ ID NO | Type | Nucleotide sequence |
|---|---|---|---|
| Cidea | 26 | Forward | GCCGTGTTAAGGAATCTGCTG |
| | 27 | Reverse | TGCTCTTCTGTATCGCCCAGT |
| Dio2 | 28 | Forward | CAGTGTGGTGCACGTCTCCAATC |
| | 29 | Reverse | TGAACCAAAGTTGACCACCAG |
| Collagen 1a | 30 | Forward | GAGCGGAGAGTACTGGATCG |
| | 31 | Reverse | GTTCGGGCTGATGTACCAGT |
| Collagen 3a | 32 | Forward | AGGCTGAAGGAAACAGCAAA |
| | 33 | Reverse | TAGTCTCATTGCCTTGCGTG |
| Sod2 | 34 | Forward | GAAGTGAAACCTCACTCACG |
| | 35 | Reverse | CAATAAAGACTACAGCACCCC |
| PGC1a | 36 | Forward | TAAGGATTTCGGTGGTGACA |
| | 37 | Reverse | TGTCACCACCGAAATCCTTA |
| 36B4 | 38 | Forward | GAAGACAGGGCGACCTGGAA |
| | 39 | Reverse | TTGTCTGCTCCCACAATGAAGC |

### Example B (5): PAS staining of the liver tissues

The liver tissue fixed with 4% PFA was deparaffinized, then reacted with 1% periodic acid aqueous solution for 10 minutes, and washed under running water for 5 minutes. Then, it was reacted with Schiff reagent for 10 minutes, reacted with 0.5% bisulfite aqueous solution, and washed under running water for 5 minutes. After the nuclear staining was carried out with Mayer's Hematoxylin solution, it was washed again under running water, dehydrated in 95% alcohol followed by washing in 100% alcohol, and then permeated and embedded.

### Example B (6): HE staining of the liver tissues

After the liver tissue fixed with 4% PFA was deparaffinized, it was immersed and reacted in a staining bottle of Hematoxylin solution for 1 to 5 minutes. After washing under running water for 5 minutes, the tissue was reacted with Eosin solution for 5 to 10 minutes and rinsed with water. Subsequently, in order to dehydrate, the tissue was immersed in 90% ethanol, followed by 95% ethanol and absolute ethanol. After immersion in xylene, it was embedded.

### Example B (7): Western blotting

As primary antibodies for insulin signaling proteins (p-IR, p-Akt, p-S6K, p-GS, p-Foxo), Phospho-Insulin Receptor β (Tyr1361) (84B2) Rabbit mAb#3023, Phospho-Akt (Ser473) Antibody#9271, Phospho-p70S6 Kinase (Thr389) Antibody#9205, Phospho-Glycogen Synthase (Ser641) Antibody#3891, Phospho-FoxOl (Thr24)/FoxO3a (Thr32) Antibody#9464 were obtained from cell signaling company, and western blotting was carried out according to the protocol enclosed in the kit.

### Example B (8): Analysis of respiratory metabolism

Using metabolic cages (ARCO-2000 series) manufactured by Arco System Inc., analysis of respiratory metabolism was performed under an optimal condition.

### Example C: Results

### Example C (1): Improvement of carbohydrate-lipid imbalance

There is an etiological mechanism that can explain the onset of diseases underlying insulin resistance resulting from a disturbed balance of carbohydrate/lipid metabolism and more significant lipogenesis than carbohydrate synthesis in obese patients (Ide T. Nature Cell Biol 2004 Apr; 6(4): 351-7). Thus, improvement in the disturbed balance of carbohydrate/lipid metabolism (carbohydrate-lipid imbalance) becomes the radical approach of treatment. The present inventors focused on the function of Ptx4 gene and measured the contents of glycogen and triglyceride in the liver of C57BL/6J mice of which Ptx4 transcription was inhibited with the use of Ptx4i adenoviral vector (pAd-Ptx4i). Consequently, the inventors found that the ratio of carbohydrates to lipids increased in the liver tissues of C57BL/6J mice by inhibiting the function of Ptx4 gene, and that carbohydrate synthesis was enhanced as compared to lipogenesis.

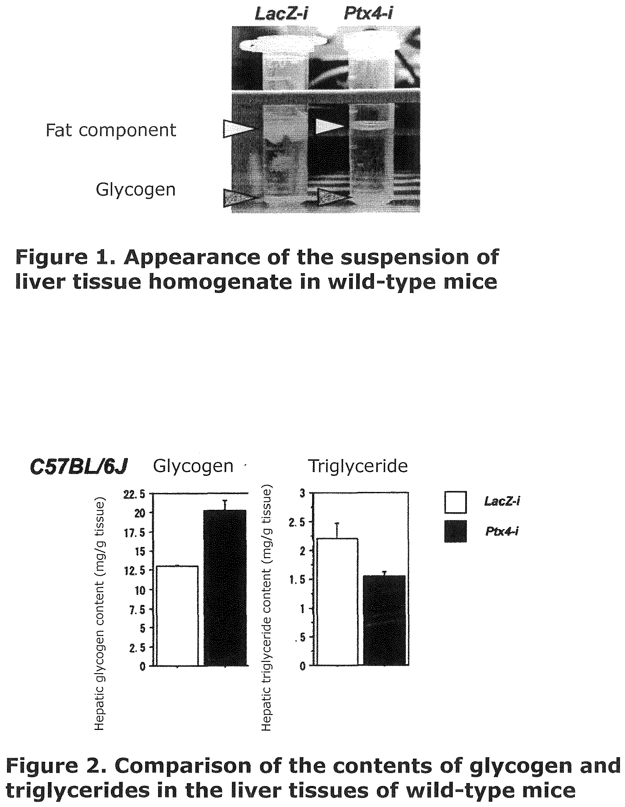

When the liver extraction solution heated in 0.6 N perchloric acid was left to stand, floating lipids were not found in the supernatant in Ptx4-inhibition (Ptx4-i) group whereas floating lipids were abundant in control (LacZ-i) group. In addition, a small amount of white precipitate was confirmed in control (LacZ-i) group, but a large amount of white precipitate was noted in the sedimentation layer in Ptx4 inhibition (Ptx4-i) group (Figure 1). When the contents of glycogen and triglyceride were measured in the liver of C57BL/6J mice, it was found that glycogen content in Ptx4-inhibition (Ptx4-i) group increased to approximately 1.6 times higher than that in control (LacZ-i) group, and triglyceride content decreased to approximately 0.68 times lower than that in control (LacZ-i) group (Figure 2).

According to these results, in the normal physiological starvation state, carbohydrate-derived energy (glycogen) is firstly utilized and thereafter, as source for fatty acid-derived energy, triglycerides produced by lipolysis from adipose tissues are utilized. When the function of Ptx4 gene was inhibited, however, it was found that triglyceride in adipose tissues or hepatocytes were first utilized as source, followed by the utilization of carbohydrate-based energy. Thus, "paradigm shift" occurred on the use of bioenergy. From the findings above, it is expected that an abnormal balance of carbohydrate/lipid metabolism where lipogenesis is dominant rather than carbohydrate synthesis in obese patients can be improved by inhibiting the function of Ptx4 gene.

### Example C (2): Enhanced lipolysis in adipose tissues and transformation into beige adipocytes

It has been known that adipose tissues are transformed into beige adipocytes having an efficient thermogenic property under a certain condition. It has also been reported that UCP1 gene expression was enhanced in beige adipocytes and the enhanced UCP1 gene expression was attributable to thermogenesis resulting in the enhancement of energy consumption (Lowell, B.B., et al. Nature 404, 652-660.2000). Accordingly, the inventors examined whether such changes in adipose tissues would occur by inhibiting the function of Ptx4 gene.

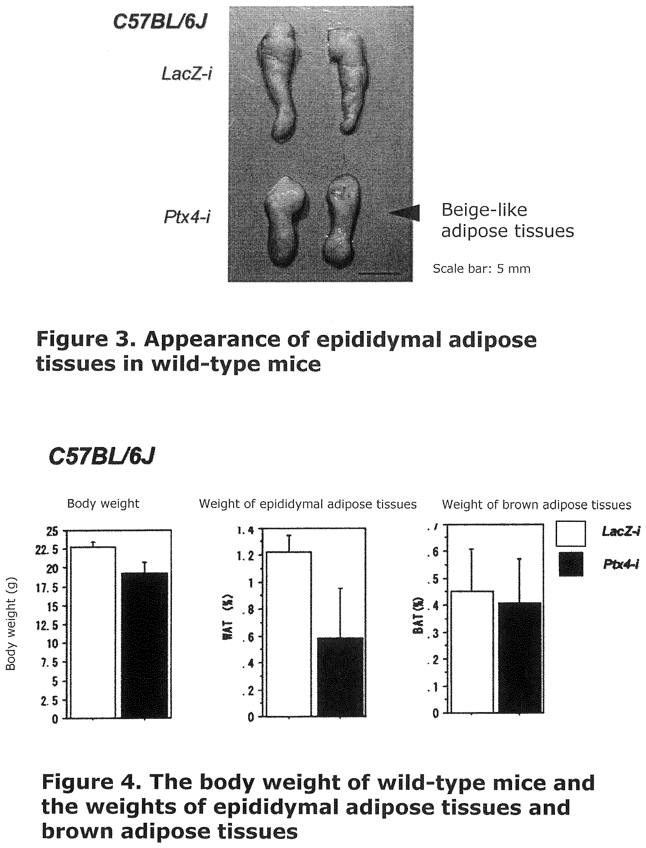

In C57BL/6J mice, the color of epididymal adipose tissues in Ptx4-inhibition (Ptx4-i) group exhibited dark reddish-brown, and was apparently different from white-like color tone seen in control (LacZ-i) group. (This is a color that is usually found in epididymal adipose tissues) (Figure 3). Additionally, it was observed that the body weight in Ptx4-inhibition (Ptx4-i) group of C57BL/6J mice decreased as compared to that in control (LacZ-i) group (Figure 4). Furthermore, it was found that the weight of epididymal adipose tissues (which are equivalent to visceral fat in human) in Ptx4-inhibition (Ptx4-i) group of C57BL/6J mice decreased by approximately 50% as compared to that in control (LacZ-i) group (Figure 4). When the gene expression of adipose tissues was measured by quantitative RT-PCR in C57BL/6J mice of which function of Ptx4 gene was inhibited, it was found that UCP1 gene expression, which was known to be enhanced in beige adipocytes, significantly increased (p<0.05).

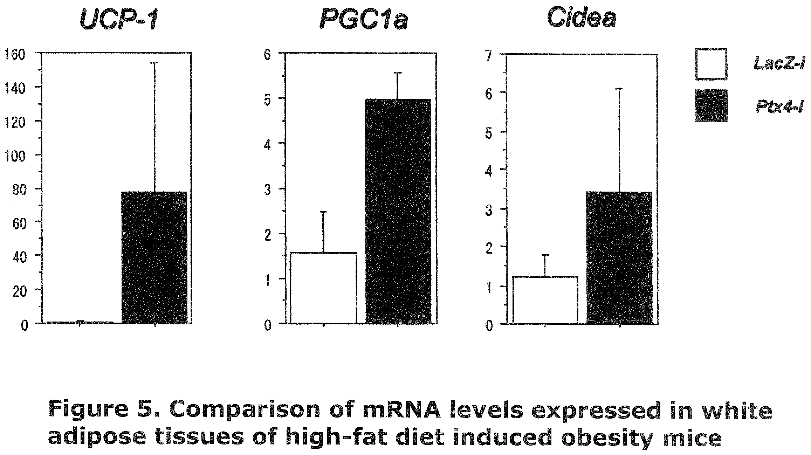

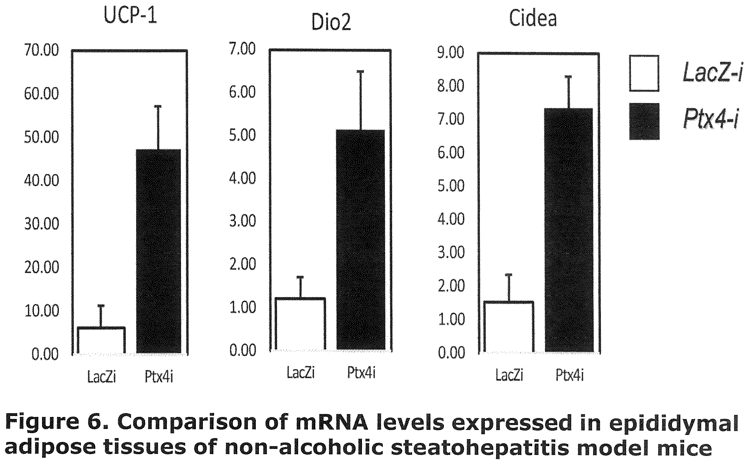

It was also confirmed that beige adipogenesis occurred in Ptx4-inhibition (Ptx4-i) group under a breeding temperature of 19°C in both high fat-diet induced obesity (Diet-induced obesity (DIO)) mice and nonalcoholic steatohepatitis (NASH) model mice (Figures 5 and 6).

It is clear from these results that lipolysis was promoted by inhibiting the function of Ptx4 gene in adipose tissues which are different from liver tissue, and that adipocytes were transformed into those having beige adipocytes-like property in which triglycerides are easily degraded and utilized. This finding seems to be caused by a similar mechanism to that of the following phenomenon: stimulation due to norepinephrine via the activation of sympathetic nerve system such as cold stimulation activates signaling cascades of p38, MAPK and PGC1α, and resulting transformation into brown adipocytes or beige adipocytes capable of activating thermogenesis (Cao, W.et al. Mol. Cell. Biol.24, 3057-3067. 2004). Because both fatty acids and glycerol released from adipose tissues account for approximately 60% with the highest contribution as sources for biosynthesis of triglyceride in the liver (Browning JD et al. J. Clin. Invest. 2004; 114(2): 147-52.), they are important factors in the metabolic regulation of triglyceride in the liver. Accordingly, it is understandable that control of lipolysis in adipose tissues induced by inhibiting the function of Ptx4 gene has a considerable impact on the metabolic regulatory system. From the findings above, it is expected that inhibition of the function of Ptx4 gene has an inhibitory effect on the accumulation of neutral fat droplets in hepatocytes, an effect of increasing lipolysis, and an anti-obesity effect.

### Example C (3): Improvement of carbohydrate/lipid balance by hepatic transcriptional regulation (decreased SREBP1 and SREBP2 expression, increased Gys2 expression or suppressed pygl expression), and anti-inflammatory response

It has been reported that insulin resistance was improved after glycogen content increased and triglyceride content decreased in the liver (Nakagawa Yet al. Nat Med. 2006 Jan; 12(1): 107-13.). Thus, the inventors examined whether hepatic transcriptional regulation leading to enhanced insulin sensitivity in the liver would occur by inhibiting the function of Ptx4 gene.

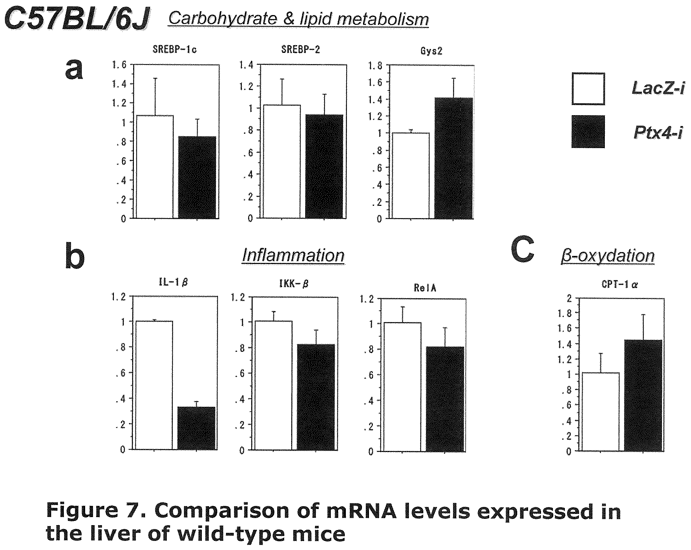

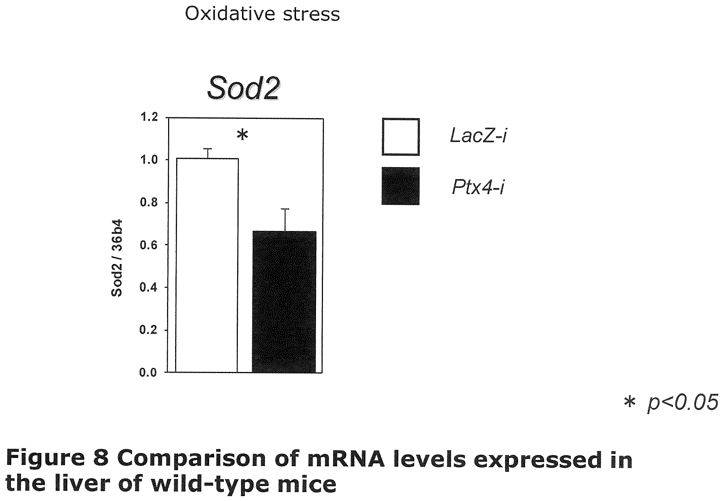

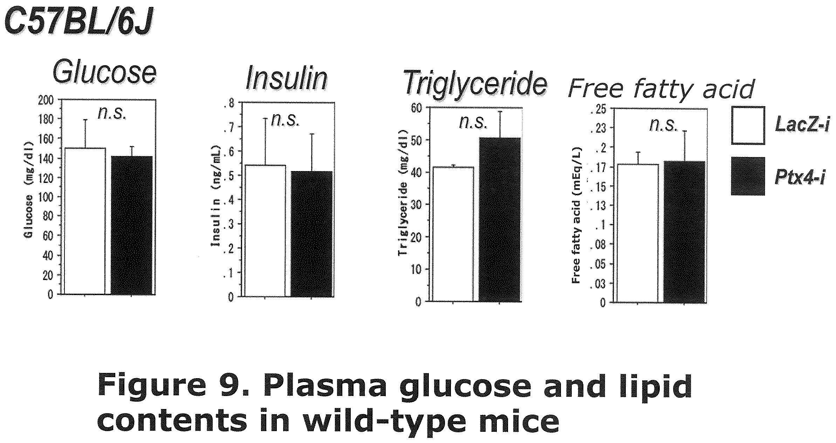

First of all, it was found that SREBP-1c and SREBP-2 genes in de novo lipogenic pathway (synthetic pathway for triglycerides and cholesterols) were suppressed in the liver tissues at the transcriptional level by inhibiting the function of Ptx4 gene (Figure 7a). On the other hand, it was also found that Gys2 gene expression responsible for glycogen synthesis was enhanced in the liver tissues at the transcriptional level by inhibiting the function of Ptx4 gene (Figure 7a). In addition, CPT-1a gene expression increased in the liver by inhibiting the function of Ptx4 gene (Figure 7c), presumed enhanced β-oxidation. Furthermore, it was found that gene expressions of inflammation-induced transcription factors IKK-β and NFκB and of inflammatory cytokine IL-1β decreased by inhibiting the function of Ptx4 gene, indicating the consistency with findings mentioned above (Figure 7b). Moreover, it was confirmed that the decreased expression of Sod2, an oxidative stress marker, and decreased oxidative stress level in hepatocytes were observed in Ptx4-inhibition (Ptx4-i) group under a breeding temperature of 19°C (Figure 8). However, there were no significant changes in plasma levels of casual glucose, insulin, triglyceride and free fatty acids even when the function of Ptx4 gene was inhibited (Figure 9).

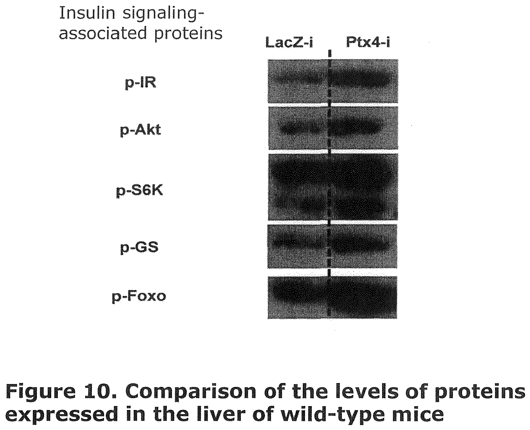

Ptx4i adenoviral vector (pAd-Ptx4i) was administered to wild-type mice (C57BL/6J) under a breeding temperature of 19°C. One week after the administration, the mice were killed, and proteins were extracted from the liver. The expression of insulin signaling-related proteins was analyzed by Western blotting. The improvement of insulin signaling was observed in Ptx4-inhibition (Ptx4-i) group, suggesting enhanced insulin sensitivity in the liver. Thus, an improving effect on insulin resistance (obesity-related inflammatory changes) was confirmed by the administration of Ptx4i adenoviral vector (pAd-Ptx4i) (Figure 10).

### Example C (4): Improvement of fatty liver due to the improvement of disturbed balance of carbohydrate/lipid (carbohydrate-lipid imbalance) and inhibitory effect on inflammation in obesity model mice

From the results of analysis of Examples (1) to (3) mentioned above in wild-type C57BL/6J mice, it was found that the inhibition of Ptx4 gene function resulted in 1) predominant utilization of hepatic fatty acids in carbohydrate/lipid balance (promoted β-oxidation), 2) decreased levels of gene expressions in de novo lipogenic pathway, 3) induced anti-inflammatory response, and 4) decreased oxidative stress. The inventors examined whether the effects similar to the above as a therapeutic effect could be confirmed in obesity model mice. As obesity model mice, high-fat diet induced obesity (Diet-induced obesity (DIO)) mice and leptin gene-deficient mice (Lep^{ob}/Lep^{ob}) were used for the following analysis.

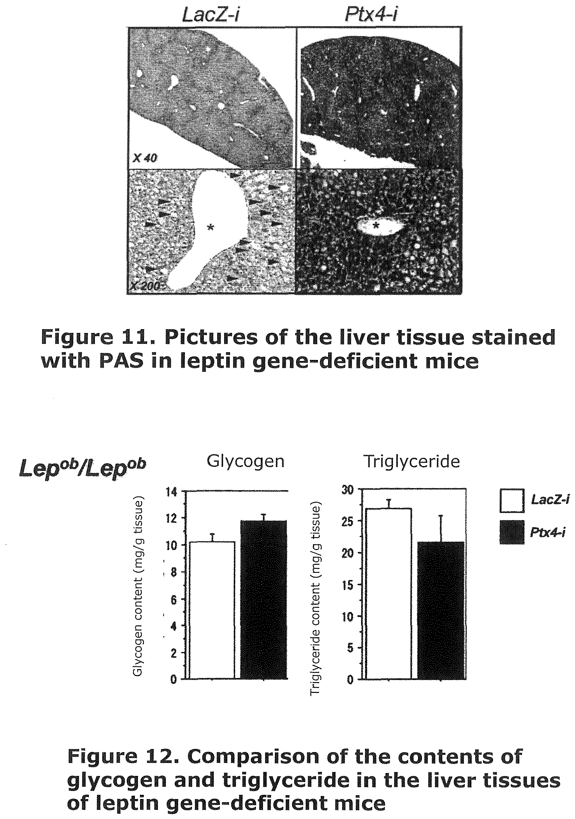

Ptx4i adenoviral vector (pAd-Ptx4i) was intravenously administered to leptin gene-deficient mice (Lep^{ob}/Lep^{ob}). PAS staining was performed for immunohistochemical analysis of the liver (Figure 11). In Figure 11, asterisk shows a central vein of the liver and arrowheads point out lipid droplets. In leptin gene-deficient mice (Lep^{ob}/Lep^{ob}), it was confirmed that many large lipid droplets were mainly accumulated around the portal vein and central vein in control (LacZ-i) group, whereas lipid droplets remarkably decreased and lipid droplets with smaller diameter were observed in Ptx4-inhibition (Ptx4-i) group (Figure 11). Furthermore, in leptin gene-deficient mice (Lep^{ob}/Lep^{ob}), it was confirmed on PAS staining that a variety of polysaccharides, having sugar chains including glycogen as a representative, increased in control (LacZ-i) group, but PAS-positive cells more intensely stained with dark blue-violet increased in Ptx4-inhibition (Ptx4-i) group (Figure 11). After these findings were quantified, it was confirmed that hepatic glycogen content increased and hepatic triglyceride content decreased by inhibiting the function of Ptx4 gene, which was similar to the results in wild-type C57BL/6J mice (Figure 12).

From these observations, it was also found that disturbed balance of carbohydrate/lipid metabolism (carbohydrate/lipid imbalance) was improved by inhibiting the function of Ptx4 gene in leptin gene-deficient mice (Lep^{ob}/Lep^{ob}).

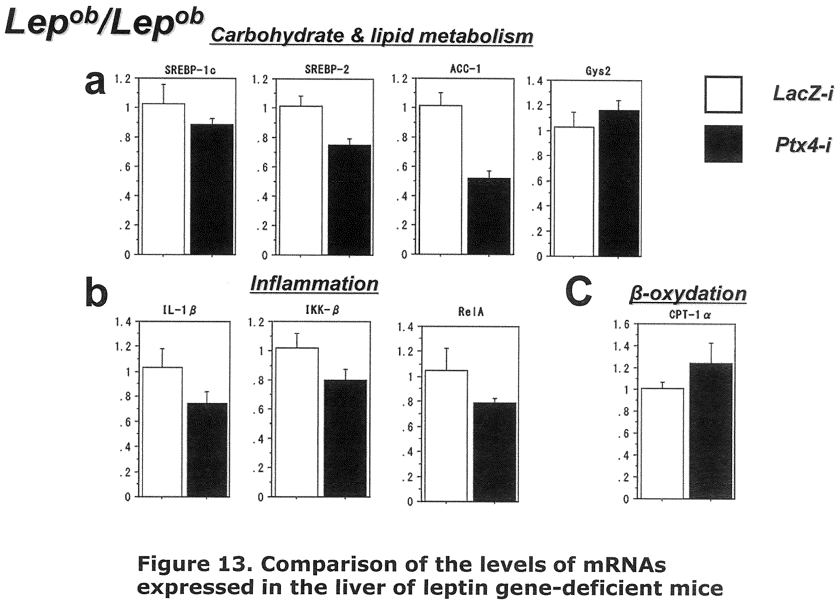

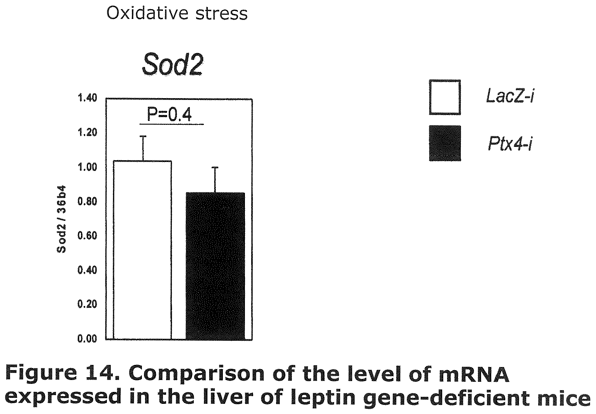

Next, it was found that in the fatty liver tissues of leptin gene-deficient mice (Lep^{ob}/Lep^{ob}), gene expression (SREBP-1c and SREBP-2) in de novo lipogenic pathway was suppressed at the transcriptional level by inhibiting the function of Ptx4 gene. On the other hand, it was found that the expression of Gys2 gene for glycogen synthesis in the fatty liver tissues was enhanced at the transcriptional level by inhibiting the function of Ptx4 gene (Figure 13a). It was confirmed that CPT-1a gene expression increased in the fatty liver tissues by inhibiting the function of Ptx4 gene, presumed enhanced β-oxidation (Figure 13c). Furthermore, it was also found that gene expressions of inflammation-induced transcription factors, IKK-β and NFκB and of inflammation cytokine IL-1β decreased by inhibiting the function of Ptx4 gene (Figure 13b). Furthermore, it was confirmed that in Ptx4-inhibition (Ptx4-i) group under a breeding temperature of 19°C, the expression of Sod2, an oxidative stress marker, decreased and oxidative stress in hepatocytes also decreased (Figure 14).

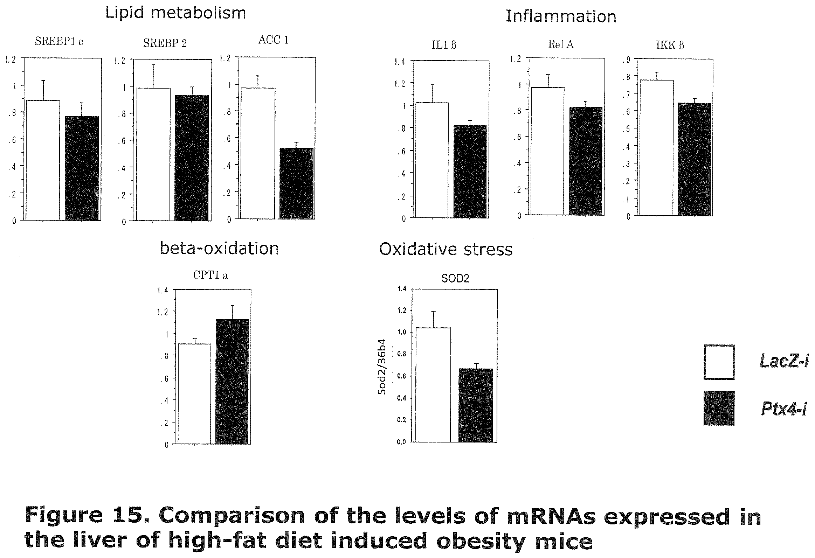

The similar pattern of gene expression was confirmed in the liver of diet-induced obesity (DIO) mice under a breeding temperature of 19°C, and thus the inhibitory effect on Ptx4 gene was confirmed with consistency in multiple obesity model mice (Figure 15).

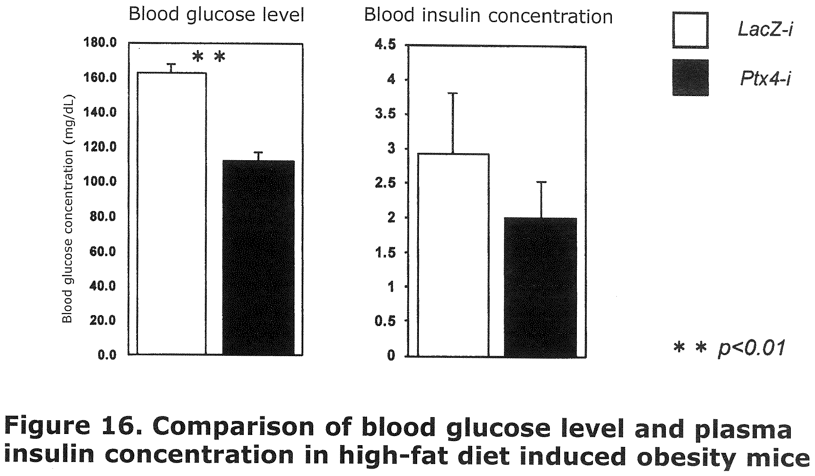

Ptx4i adenoviral vector (pAd-Ptx4i) was administered to diet-induced obesity (DIO) mice under a breeding temperature of 19°C. One week after the administration, both blood casual glucose and plasma insulin concentrations were measured using an ELISA method. Consequently, a remarkable reduction in blood glucose level and a tendency to decrease in blood insulin level were observed in Ptx4-inhibition (Ptx4-i) group (Figure 16).

### Example C (5): Proof-of-concept of therapeutic agents for non-alcoholic steatohepatitis (NASH)

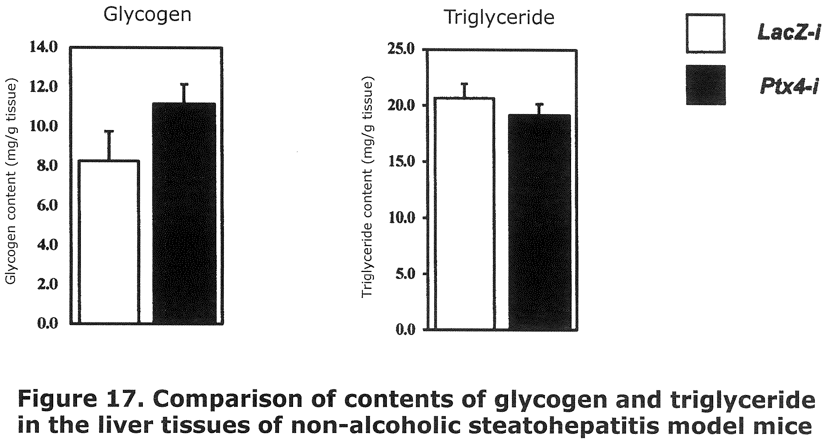

From the above, it was expected that a prophylactic or therapeutic effect is exerted by inhibition of the function of Ptx4 gene on non-alcoholic steatohepatitis (NASH) accompanied by neutral fat accumulation. After mice were fed with choline-deficient, methionine-defined, high-fat diet (CDAHFD) for three weeks to produce NASH model mice (CDAHFDNASH model mice), Ptx4i adenoviral vector (pAd-Ptx4i) was administered. One week after the administration of the vector, the contents of triglyceride and glycogen in the liver were measured. All breeding temperatures were set to 19°C. Consequently, a tendency to increase in glycogen content and a tendency to decrease in triglyceride content were observed in Ptx4-inhibition (Ptx4-i) group of NASH model mice (CDAHFD NASH model mice) (Figure 17).

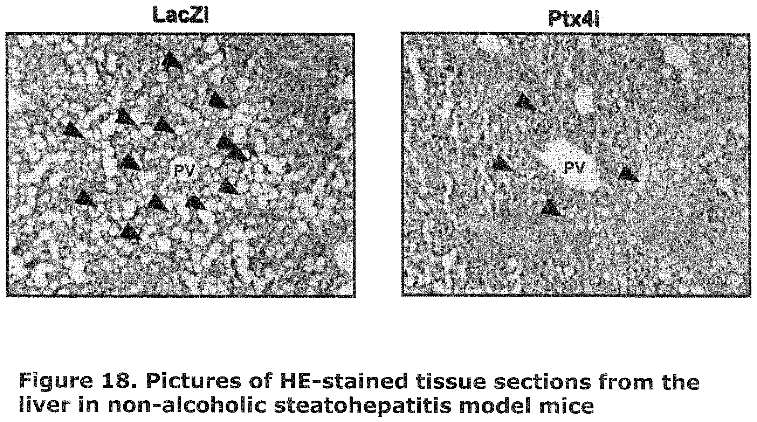

Ptx4i adenoviral vector (pAd-Ptx4i) was administered to the NASH model mice mentioned above under a breeding temperature of 19°C, and one week after the administration, histological examination of the liver was performed. As a result, a decrease in areas of void regions around the portal vein (PV) (decreased number of lipid droplets) was observed in Ptx4-inhibition (Ptx4-i) group, suggesting improvement of the fatty liver, a pathological index for NASH (Figure 18).

Next, mRNA expression levels of superoxide dismutase 2 (Sod2), one of indices of anti-oxidative stress in the liver, were investigated in order to examine anti-oxidative responses by inhibiting the function of Ptx4 gene in each model mouse. As a result, a significant decrease and tendency to decrease in Sod2 mRNA expression was observed in Ptx4-inhibition (Ptx4-i) group of wild-type mice, diet induced obesity (DIO) mice, and NASH model mice (CDAHFD NASH model mice) (Figures 8, 15 and 19). These results suggested that oxidative stress in the liver was alleviated by the administration of Ptx4i adenoviral vector (pAd-Ptx4i), leading to decrease in mRNA expression levels in NASH model mice.

Furthermore, mRNA expression levels of Collagenla and Collagen3a were investigated to confirm an effect by inhibition of the function of Ptx4 gene on hepatic fibrosis in mice with non-alcoholic steatohepatitis (NASH). In mice with NASH, a decrease in the gene expression for each hepatic fibrosis marker was observed in Ptx4-inhibition (Ptx4-i) group, and it was also confirmed that exacerbated hepatic fibrosis was improved by the administration of Ptx4i adenoviral vector (pAd-Ptx4i) (Figure 19).

The above-mentioned results are therapeutic outcome satisfying all the criteria for potential endpoints stated in Proof-of-Concept on therapeutic agents for non-alcoholic steatohepatitis (NASH) approved by Food and Drug Administration (FDA) as the following: (i) a decrease in liver fat contents, and (ii) a decrease in expression levels of cell damage markers (for inflammation, oxidative stress, and fibrosis).

Sources for intrahepatic fat include the following: i) free fatty acids from adipose tissues (60%), ii) de novo lipogenesis (26%), and iii) derivings from glucose or fat ingested (14%) (Kerry L. et al. J Clin Invest. 2005; 115 (5): 1343-1351. doi: 10.1172/JCI23621). Accordingly, the inventors examined whether the amount of glucose or fat ingested would be reduced by inhibiting the function of Ptx4 gene in leptin gene-deficient mice (Lep^{ob}/Lep^{ob}).

Consequently, decrease in both oral food consumption and body weight was confirmed by inhibiting the function of Ptx4 gene in leptin gene-deficient mice (Lep^{ob}/Lep^{ob}) (Figure 20).

It was clear from the above results that decrease in food consumption and weight loss by inhibiting the function of Ptx4 gene were effective for obesity as a pathological condition. PPARγ agonists and vitamin E are known as active ingredients of preexisting therapeutic drugs for non-alcoholic steatohepatitis (NASH) (Hardy T. et al. Current Opinion in Gastroenterology 31(3): 175-183. 2015). However, an active ingredient has not been discovered yet, which has an effect on all the three fat sources involved in liver fat accumulation described above. Accordingly, it is expected for the present invention to have a clinical effect as a novel drug exerting a new effect on non-alcoholic steatohepatitis (NASH).

### Example C (6): Organ protection and disease prevention by metabolic control in a false hibernation state

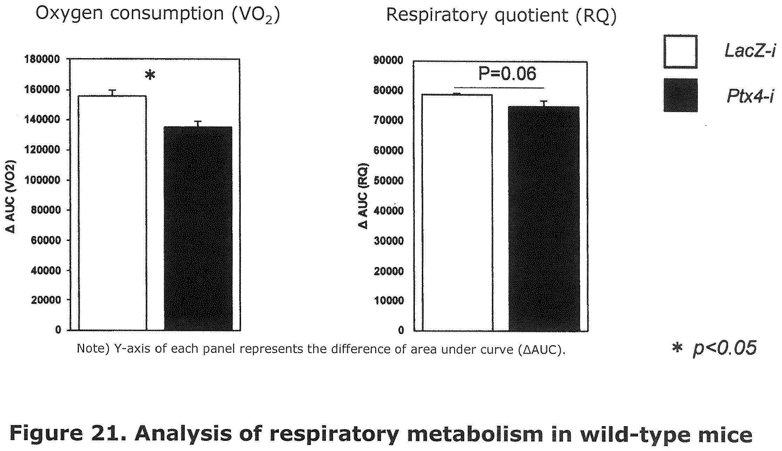

Twenty-four hours after Ptx4i adenoviral vector (pAd-Ptx4i) was administered to wild-type mice (C57BL/6J) under a breeding temperature of 19°C, oxygen consumption (VO₂) and respiratory quotient (RQ) were measured using metabolism cages. A graph was created from the results and area under the curve (AUC) was calculated. Consequently, it was confirmed that oxygen consumption (VO₂) significantly decreased, indicating of decreased metabolic response, and respiratory quotient (RQ) also decreased in Ptx4-inhibition (Ptx4-i) group of wild-type mice (C57BL6/J) (Figure 21).

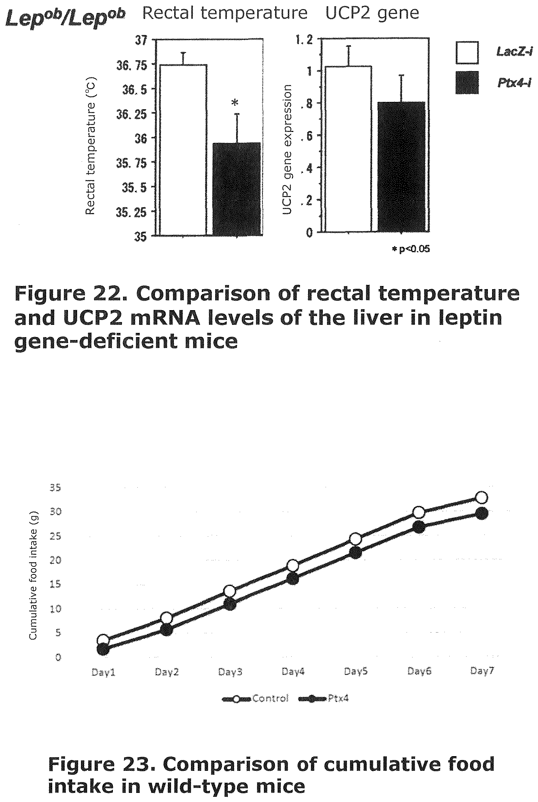

In leptin gene-deficient mice (Lep^{ob}/Lep^{ob}), it was found that rectal temperature (deep body temperature) in Ptx4-inhibition (Ptx4-i) group dropped by approximately 0.9°C as compared to that of control (LacZ-i) group. It was also found that UCP2 gene expression decreased in the liver (Figure 22).

Although a mechanism that can explain this phenomenon has not been fully elucidated yet, hibernation and torpor have apparent physiological conditions such as decreased physiological level of metabolism and decreased body temperature. It has also been reported that hibernation-specific protein (HP), which is a molecule secreted from the liver, acted directly on the brain to induce hibernation (Kondo N et al. Cell. 2006 Apr 7; 125(1): 161-72).

Similarities are seen between physiological findings caused by hibernation and those induced by inhibiting the function of Ptx4 gene in the present invention. They include the reduction of basal metabolism, decrease in body temperature and utilization of fatty acids as dominant energy source. From these consistent findings, it is presumed that a living organism transitions to a metabolic state of false hibernation by inhibiting the function of Ptx4 gene. Thus, these findings are important as providing a view that unravels an essential physiological role of pentraxin-4 protein encoded by Ptx4 gene.

In addition, it has been reported that hibernation has an effect of protecting organs from diseases or disorders induced by various adverse events. Such effects include the maintenance of lower body temperature (Hochachk et al. Science, 231 (1986), 234-241), protection from ischemia (K.U. Frerichs. et al. J. Cereb. Blood Flow Metab., 18 (1998), pp. 168-175), protection from amyotrophy (H.J. Harlowet al. Nature, 409 (2001), p. 997), suppression of bacterial infection (V.M. Sharapov. et al. Mycopathologia, 84 (1983), pp. 77-80), and suppression of tumorigenesis (G.B. Kemper et al. Comp. Biochem. Physiol., 73C (1982), pp. 445-450) as characteristics of metabolic state in hibernation.

Thus, it is expected that metabolic state in false hibernation possibly triggered by inhibiting the function of Ptx4 gene is effective for organ protection such as protection from hepatic cirrhosis or malignancy due to non-alcoholic steatohepatitis (NASH) or complications due to diabetes mellitus. Furthermore, because of decreased basal metabolism and alleviation of cell damage induced by radiation exposure, the present invention is expected to promote defensive responses against long-term exposure to cosmic rays, and be applied to space business development (Cerri M et al. Life Sci Space Res., 90(4) (2016) pp.445-452).

### Example D (1): Preparation of antibody recognizing pentraxin-4 protein

In order to prepare an antibody that recognizes pentraxin-4 protein, anti-pentraxin-4 rabbit IgG polyclonal antibody was prepared with the peptide of Amino acid sequence 2 (SEQ ID NO 4) described in Table 2 using antibody contract manufacturing service provided by Eurofins Genomics K.K.

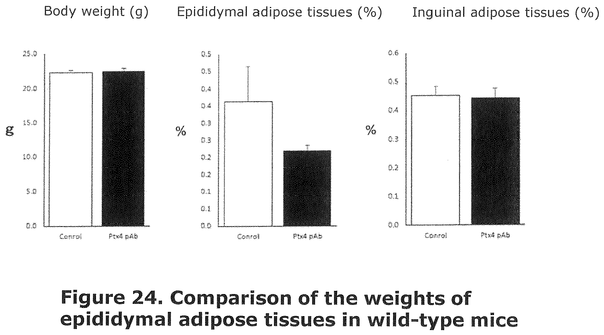

Example D (2) Inhibitory effect by antibody recognizing pentraxin-4 protein on pentraxin-4 protein function An inhibitory effect of an anti-pentraxin-4 rabbit IgG polyclonal antibody on pentraxin-4 protein function *in vivo* was examined. An anti-pentraxin-4 rabbit IgG polyclonal antibody (50 µg/body) and a rabbit IgG antibody (50 µg/body) as control were each intravenously administered to wild-type mice (C57BL/6J) under a breeding temperature of 19°C. During 7 days after administration, the inhibitory effects of these antibodies on pentraxin-4 protein function were investigated. Consequently, the decrease in cumulative food consumption was observed since the administration in the experimental group receiving anti-pentraxin-4 rabbit IgG polyclonal antibody (Figure 23). In the experimental group receiving anti-pentraxin-4 rabbit IgG polyclonal antibody, a tendency to decrease sharply in the weight of epididymal adipose tissues was observed as compared to that of inguinal adipose tissues, suggesting improved insulin resistance (Figure 24). It was confirmed from these results that loss of appetite and a strong tendency to reduce the weight of epididymal adipose tissues were observed, and pentraxin-4 protein function was inhibited by the administration of this antibody, which is similar to the results observed after the administration of Ptx4i adenoviral vector (pAd-Ptx4i). From the above, it was shown that the administration of antibody recognizing pentraxin-4 protein can be effective for improving the conditions of obesity-related diseases.

## Claims

1. A method for screening a drug for treating obesity-related diseases **characterized by** using an ability of a candidate drug to inhibit the action of pentraxin-4 as an index, and by identifying a candidate drug which has the ability as a drug for treating obesity-related diseases.

2. The method according to claim 1, wherein the index is an ability to inhibit the expression of pentraxin-4 gene.

3. The method according to claim 2, wherein the index is an ability to inhibit the initiation of transcription from pentraxin-4 gene to mRNA, an ability to degrade mRNA from pentraxin-4gene, or an ability to inhibit translation from the pentraxin-4 mRNA to protein.

4. The method according to claim 2 or 3, **characterized by** using a nucleic acid molecule in which a DNA fragment comprising the transcriptional regulatory region of pentraxin-4 gene and a DNA fragment comprising a reporter gene are operably ligated.

5. The method according to claim 1, wherein the index is an ability to inhibit the function of pentraxin-4 protein.

6. The method according to claim 5, wherein the index is an ability to inhibit the extracellular secretion of the pentraxin-4 protein, an ability to inhibit the transfer of the pentraxin-4 protein into neurons or an ability to inhibit signal transduction from the pentraxin-4 protein.

7. The method according to claim 5 or 6, comprising a step of selecting a substance which specifically binds to pentraxin-4 protein as a primary candidate.

8. The method according to any one of claims 1 to 7, wherein a drug to be screened is a drug for improvement of obesity due to enhanced lipolysis, improvement of insulin resistance, anti-obesity due to beige adipogenesis, decreased liver fat accumulation, decreased hepatic inflammation, anti-oxidative stress, or hepatic anti-fibrotic response.

9. The method according to any one of claims 1 to 8, wherein the obesity-related disease is non-alcoholic steatohepatitis (NASH) or diabetes mellitus.

10. A pharmaceutical composition comprising a pentraxin-4 inhibitor, for treating obesity-related diseases.

11. The pharmaceutical composition according to claim 10, wherein the pentraxin-4 inhibitor is an RNAi mediator which specifically inhibits the expression of pentraxin-4 gene.

12. The pharmaceutical composition according to claim 10, wherein the pentraxin-4 inhibitor is a vector expressing an RNAi mediator which specifically inhibits the expression of pentraxin-4 gene.

13. The pharmaceutical composition according to claim 10, wherein the pentraxin-4 inhibitor is an antibody which specifically binds to pentraxin-4 protein.

14. The pharmaceutical composition according to claim 10, wherein the pentraxin-4 inhibitor is an antibody which recognizes a peptide of amino acid sequence as set forth in SEQ ID NO 4.

15. The pharmaceutical composition according to claim 13 or 14, wherein the antibody is a monoclonal antibody.

16. The pharmaceutical composition according to any one of claims 10 to 15, for improvement of obesity due to enhanced lipolysis, improvement of insulin resistance, anti-obesity due to beige adipogenesis, decreased liver fat accumulation, decreased hepatic inflammation, anti-oxidative stress, and hepatic anti-fibrotic response.

17. The pharmaceutical composition according to any one of claims 10 to 16, wherein the obesity-related disease is non-alcoholic steatohepatitis (NASH) or diabetes mellitus.

18. A pentraxin-4 inhibitor for use in therapy.

19. A pentraxin-4 inhibitor for use in the treatment of obesity-related diseases.

20. A use of a pentraxin-4 inhibitor for the manufacture of a medicament for the treatment of obesity-related diseases.

21. A method for treating obesity-related diseases comprising administering a therapeutically effective dose of pentraxin-4 inhibitor to a subject in need thereof.
